# EUROPEAN PATENT APPLICATION

(11) **EP 3 858 818 A1**
(43) Date of publication of application: **04.08.2021**
(21) Application number: 19865076.4
(22) Date of filing: 29.09.2019
(51) Int. Cl.: C07D 239/96, C07D 239/95, A01N 43/54, A01P 13/00

(54) **TRIKETONE COMPOUND, PREPARATION METHOD THEREFOR AND USE THEREOF, AND HERBICIDE**

(30) Priority: 29.09.2018 CN 201811150620
(71) Applicant: Shandong Cynda Chemical Co., LTD., Shandong 256500 (CN)
(72) Inventor: YANG, Guangfu, Wuhan, Hubei 430079 (CN); QU, Renyu, Wuhan, Hubei 430079 (CN); WANG, Xianquan, Binzhou, Shandong 256500 (CN); CHEN, Enchang, Binzhou, Shandong 256500 (CN); ZHANG, Tianzhu, Binzhou, Shandong 256500 (CN); DU, Chen, Binzhou, Shandong 256500 (CN)
(74) Representative: Ström & Gulliksson AB
(86) International application number: PCT/CN2019/109175
(87) International publication number: WO 2020/063981

(57) **Abstract**

A triketone compound, a preparation method therefor and the use thereof, and a herbicide are disclosed. The compound has a structure represented by formula (I). The triketone compound can be used as an HPPD herbicide for controlling gramineous weeds and broadleaf weeds. The triketone compound has good control effects on various gramineous weeds and some broadleaf weeds which are harmful to wheat and/or peanut growth.

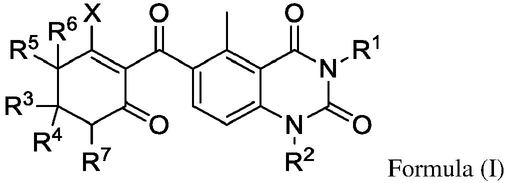

## Description

### Field of the Invention

The invention relates to the field of pesticide herbicides, in particular to a triketone compound, a preparation method and use thereof, and a herbicide containing the triketone compound.

### Background of the Invention

The explosive growth of resistance of weeds for herbicide has become a key challenge for the sustainable development of modern agriculture. The development of novel ultra-efficient herbicides is a fundamental solution to cope with this challenge.

At present, more than 40 species of resistant weeds are officially reported in China, and China is one of the five countries most threatened by resistant weeds in the world. Particularly, in several important crop fields in China, the problem of resistance of weeds for herbicide has shown a more and more serious trend. For example, some resistant weeds in wheat fields such as *Descurainia sophia, Capsella bursa-pastoris, Alopecurus aequalis, Alopecurus japonicus, Bromus japonicus, Aegilops squarrosa,* and *Avena fatua* have been developed into dominant populations. As a result, the application amount of herbicides in wheat fields in some provinces and cities is continuously increased, which not only increases the control cost but also leads to frequent phytotoxicity incidents and excessive pesticide residues.

Therefore, under the current background that "reduction synergism and green development" are vigorously advocated in the country, the development of ultra-efficient herbicides with novel mechanisms of action to replace traditional herbicides is the fundamental way to control resistant weeds and realize reduction synergism and green development.

Herbicides targeting *p*-hydroxyphenylpyruvate dioxygenase (HPPD) are widely used due to their high efficacy, low toxicity, environmental friendliness, and low resistance risk.

Up to now, dozens of herbicides targeting HPPD were developed successfully, and they can be classified roughly into three major classes according to structural classification: 1. triketones represented by Mesotrione, 2. pyrazoles represented by Topramezone, 3. isoxazoles represented by Isoxachlorotole.

According to the investigation on pesticide market sales, the time of the HPPD inhibiting herbicides entering into the market is later than that of AHAS-, PPO-and ACCase-inhibiting herbicides, but it does not hinder them to become the "star molecules" in herbicide fields and occupy the market rapidly. Particularly, the triketone herbicide-Mesotrione developed by Syngenta company occupies continuously the top five of herbicide sales for many years. Mesotrione has replaced other types of herbicides with a high incidence of resistant weeds gradually in corn fields and has become the most widely used and effective herbicide in corn fields.

However, Mesotrione still has some self-disadvantages. It has not only poor safety for other main crops and industrial crops such as wheat, rice, peanut, soybean, and rape but also poor control effect to various gramineous weeds (such as *Setaria viridis* and *Setaria glauca*)*.* There are also reported in the literature that weeds are prone to turn green after treated by Mesotrione. Cypyrafluone created by KingAgroot company becomes the first selective HPPD herbicide used for controlling weeds in wheat fields in China (as disclosed in CN105218449A), which can effectively control resistant and multi-resistant *Alopecurus aequalis, Alopecurus japonicus,* and other gramineous weeds, as well as some broadleaf weeds. Cypyrafluone provides more choices for the use of herbicides in wheat fields. However, Cypyrafluone is not ideal in controlling multiple gramineous weeds that endanger the growth of wheat, such as *Bromus japonicus, Avena fatua,* and *Aegilops squarrosa.* The specific structure of Cypyrafluone as follows:

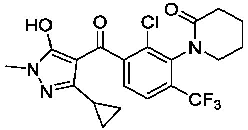

In view of the fact that there is no HPPD herbicide which has broad-spectrum and ultra-efficient weed-controlling activity in wheat fields in agricultural production in China. And there is especially no HPPD herbicide that has an excellent control effect toward multiple gramineous weeds which endanger the growth of wheat, such as *Bromus japonicus, Avena fatua,* and *Aegilops squarrosa.* In addition, for the economic crop peanuts, there is still a lack of available HPPD-inhibiting herbicides that can be used to control gramineous weeds in peanut fields, such as *Echinochloa crusgalli, Digitaria sanguinalis,* and *Setaria viridis.* Therefore, the discovery of novel ultra-efficient HPPD herbicides which are safe for wheat and peanut is of great significance in meeting the practical requirements in agricultural production in China.

### Summary of the Invention

The invention aims to overcome the above-mentioned drawbacks in the prior art by providing an HPPD herbicide that can be used for controlling weeds in wheat and/or peanut fields.

The invention also aims to provide a triketone compound that has good control effects against various gramineous weeds and some broadleaf weeds that are harmful to wheat and/or peanut growth, such as *Echinochloa crusgalli, Digitaria sanguinalis, Setaria viridis, Bromus japonicus, Avena fatua,* and *Aegilops squarrosa* and others.

In order to achieve the above-mentioned aim, the first aspect of the invention provides a triketone compound having a structure represented by formula (I),

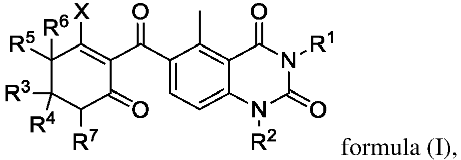

Wherein, in the formula (I),
R¹ is *n*-propyl or cyclopentyl;
R² is selected from H, C₁₋₆ alkyl, C₁₋₃alkyl substituted by 1 to 6 halogen atoms, C₂₋₆ alkynyl, C₅₋₈alkynyl substituted by trimethylsilyl, C₂₋₆ alkenyl, C₂₋₆ alkenyl substituted by 1-6 halogen atoms;
X is selected from hydroxyl, halogen, C₁₋₃ alkylthio, phenylthio, C₁₋₃ alkyl sulfuryl, and phenyl sulfuryl;
R³, R⁴, R⁵, R⁶, and R⁷ are selected independently from H and C₁₋₃ alkyl.

In this research, the inventor of the invention finds that triketone compounds with the general formula structure shown in the formula (I) have high safety for crops (such as wheat and peanut), and it has obvious better control effect against multiple gramineous weeds and some broadleaf weeds which are harmful to wheat and/or peanut growth, such as *Bromus japonicus, Avena fatua,* and *Aegilops squarrosa,* compared with the prior art.

The second aspect of the invention provides a method for preparing the aforementioned triketone compounds, which comprises:
(1) Reacting the compounds with the structure shown in the formula (II-1) with sulfoxide chloride and the compounds with the structure shown in the formula (II-2) in sequence to obtain the compounds with the structure shown in the formula (II-3);
(2) Under the rearrangement reaction condition, the compounds with the structure shown in the formula (II-3) is contacted with the catalysts in the presence of alkalis and solvents to obtain the compounds with the structure shown in the formula (II-4);

Optionally, the method further comprises 1, 2, or 3 of the following steps in sequence:
(a) Carrying out halogenation reaction on the compounds with the structure shown in the formula (II-4) to obtain the compounds with the structure shown in the formula (II-5);
(b) Reacting the compounds with the structure shown in the formula (II-5) with sodium mercaptides with the structure shown in the formula (II-6) to obtain the compounds with the structure shown in the formula (II-7);
(c) Carrying out oxidation reaction on the compounds with the structure shown in the formula (II-7) in the presence of peroxides with the structure shown in the formula (II-8) to obtain the compounds with the structure shown in the formula (II-9);

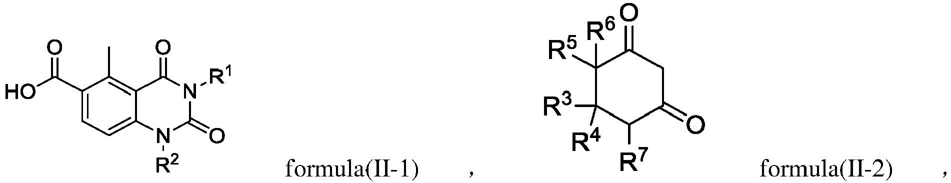

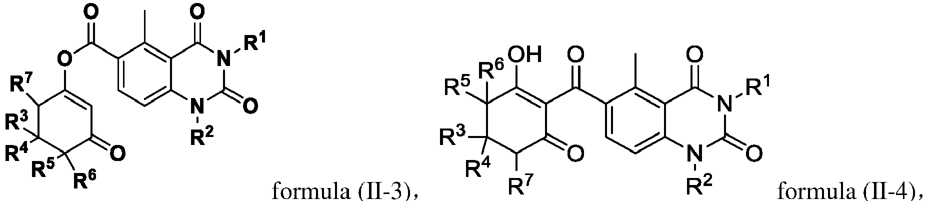

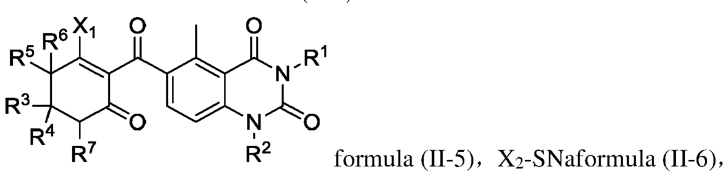

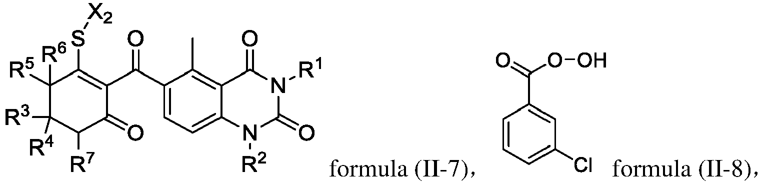

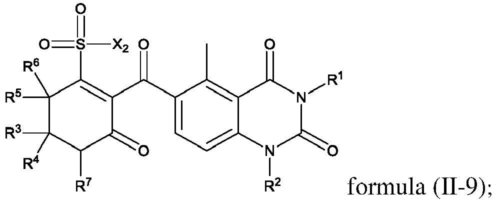

Wherein,
X₁ in the compounds with the structure shown in the formula (II-5) is halogen;
X2 in the compounds with the structure shown in the formula (II-6), the formula (II-7) and the formula (II-9) is selected from C₁₋₃alkyl and phenyl;
moreover, the remaining substituents involved in the formula (II-1), the formula (II-2), the formula (II-3), the formula (II-4), the formula (II-5), the formula (II-7), and the formula (II-9) are the same as those of the aforementioned compounds of the invention.

The third aspect of the invention provides the use of the aforementioned triketone compounds for controlling weeds.

The fourth aspect of the invention provides a herbicide, which comprises active ingredients and adjuvants, wherein, the active ingredients comprises one at least of the aforementioned triketone compounds.

The triketone compounds provided by the invention can be used as HPPD herbicides for wheat and/or peanut crops, and they have good control effect on various gramineous weeds and some broadleaf weeds which are harmful to wheat and/or peanut growth, such as *Echinochloa crusgalli, Digitaria sanguinalis, Setaria viridis, Bromus japonicus, Avena fatua, Aegilops squarrosa,* and others. The triketone compounds provided by the invention also have high safety for crops.

### Detailed Description of the Embodiments

The endpoints of the ranges and any values disclosed herein are not limited to the precise range or value, and these ranges or values should be understood to encompass values close to these ranges or values. For numerical ranges, between the endpoints of each range, between endpoint values of each range and individual point values, and individual point values can be combined with each other to give one or more new numerical ranges, and these numerical ranges should be construed as specifically disclosed herein.

As mentioned above, the first aspect of the invention provides a triketone compound having a structure represented by formula (I),

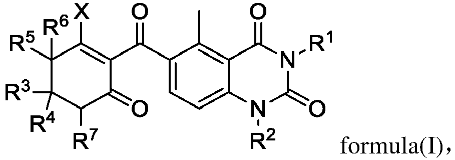

Wherein, in the formula (I),
R¹ is *n*-propyl or cyclopentyl;
R² is selected from H, C₁₋₆alkyl, C₁₋₃alkyl substituted by 1 to 6 halogen atoms, C₂₋₆ alkynyl, C₅₋₈ alkynyl substituted by trimethylsilyl, C₂₋₆ alkenyl, C₂₋₆ alkenyl substituted by 1-6 halogen atoms;
X is selected from hydroxyl, halogen, C₁₋₃ alkylthio, phenylthio, C₁₋₃ alkyl sulfuryl, and phenyl sulfuryl;
R³, R⁴, R⁵, R⁶, and R⁷ are selected independently from H and C₁₋₃alkyl.

"C₁₋₆ alkyl" refers to alkyl groups having a total number of carbon atoms of 1 to 6, including straight-chain alkyl, branched-chain alkyl, and cyclic alkyl. For example, the total number of carbon atoms of straight-chain alkyl, branched-chain alkyl, and cyclic alkyl maybe 1, 2, 3, 4, 5, or 6, such as methyl, ethyl, *n*-propyl, isopropyl, cyclopropyl, *n*-butyl, *iso*-butyl, *tert-*butyl*,* cyclobutyl, n-pentyl, isopentyl, cyclopentyl, *n*-hexyl, cyclohexyl, and others.

"C₁₋₃ alkyl" refers to alkyl groups having a total number of carbon atoms of 1 to 3, including straight-chain alkyl, branched-chain alkyl, and cyclic alkyl. For example, the total number of carbon atoms of straight-chain alkyl, branched-chain alkyl, and cyclic alkyl maybe 1, 2, or 3, such as methyl, ethyl, *n*-propyl, *iso*-propyl, and cyclopropyl.

"C₁₋₃ alkyl substituted by 1 to 6 halogen atoms" refers to alkyl groups having a total number of carbon atoms of 1 to 3, including straight-chain alkyl, branched-chain alkyl, and cyclic alkyl, for example, the total number of carbon atoms of straight-chain alkyl, branched-chain alkyl, and cyclic alkyl maybe 1, 2 or 3, and 1 to 6H on the alkyl are substituted by halogen atoms, such as 1, 2, 3, 4, 5, or 6H may be substituted by one at least halogen atoms selected from fluorine, chlorine, bromine, and iodine.

"C2-6 alkynyl " refers to alkynyl groups having a total number of carbon atoms of 2 to 6, such as ethynyl, propynyl, butynyl, and the like.

"C₅₋₈ alkynyl substituted by trimethylsilyl" refers to alkynyl groups having a total number of carbon atoms of 5 to 8, and one at least H on the alkynyl are substituted by trimethylsilyl.

"C2-6 alkenyl" refers to alkenyl groups having a total number of carbon atoms of 2-6, such as ethenyl, propenyl, butenyl, and the like. The definition of "C2-6 alkenyl substituted by 1 to 6 halogen atoms" is similar to that of "C2-6 alkenyl", the difference is that 1 to 6H of "C2-6 alkenyl substituted by 1 to 6 halogen atoms" are substituted by halogen atoms, for example, 1, 2, 3, 4, 5 or 6H may be substituted by one at least halogen atoms selected from fluorine, chlorine, bromine, and iodine.

"C₁₋₃ alkylthio" refers to a sulfur atom having one end bonded to the parent nucleus and the other end bonded to C₁₋₃ alkyl."phenylthio" refers to a sulfur atom having one end bonded to the parent nucleus and the other end bonded to phenyl. "C₁₋₃ alkyl sulfone" refers to sulfone bonded to the parent nucleus and C₁₋₃ alkyl, respectively, "phenyl sulfone" refers to sulfone bonded to the parent nucleus and phenyl, respectively.

According to a preferred embodiment, in the formula (I),
R¹ is *n*-propyl or cyclopentyl;
R² is selected from H, C₁₋₃ alkyl, C₁₋₃ alkyl substituted by 1 to 3 halogen atoms, C₃₋₆ alkynyl, C₆₋₈ alkynyl substituted by trimethylsilyl, C₃₋₆ alkenyl, C₃₋₆ alkenyl substituted by 1-3 halogen atoms;
X is selected from hydroxyl, halogen, C₁₋₃ alkylthio, phenylthio, C₁₋₃ alkyl sulfuryl, and phenyl sulfuryl;
R³, R⁴, R⁵, and R⁶ are selected independently from H and C₁₋₃ alkyl;
R⁷ is H.

According to another preferred embodiment, in the formula (I),
R¹ is *n*-propyl or cyclopentyl;
R² is selected from H, methyl, ethyl, monofluoromethyl, difluoromethyl, monofluoroethyl, difluoroethyl, trifluoromethyl, trifluoroethyl, 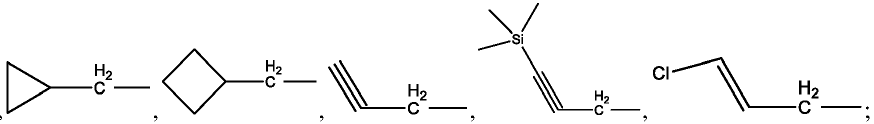 X is selected from-OH, -F,-Cl,-Br,CH₃-S-,CH₃CH₂-S-,CH₃CH₂CH₂-S-, 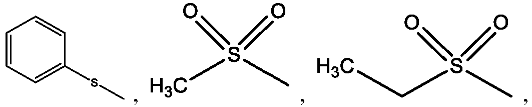 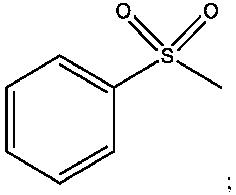 R³, R⁴, R⁵, and R⁶ are selected independently from H, methyl, ethyl, *n*-propyl and isopropyl;
R⁷ is H.

According to a particularly preferred embodiment, the compound represented by formula (I) are at least one selected from the followings:

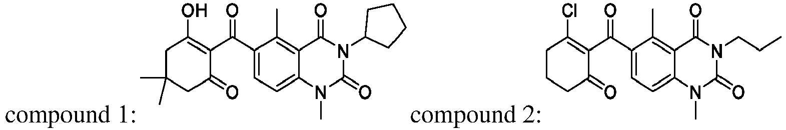

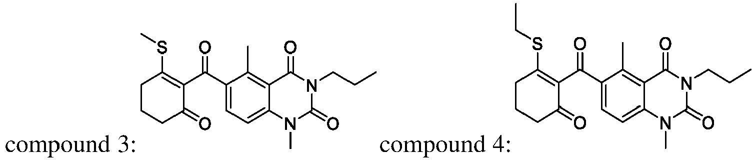

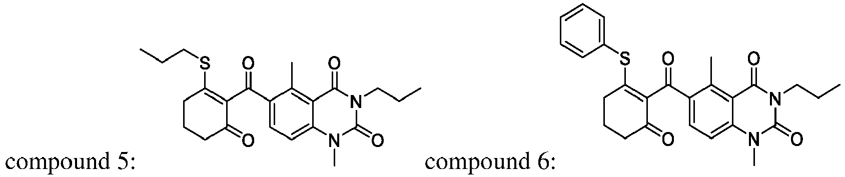

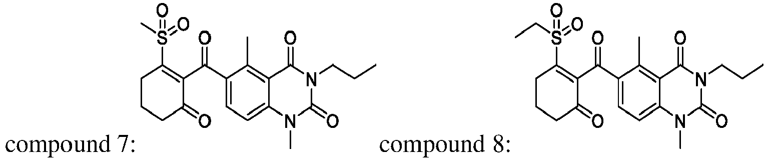

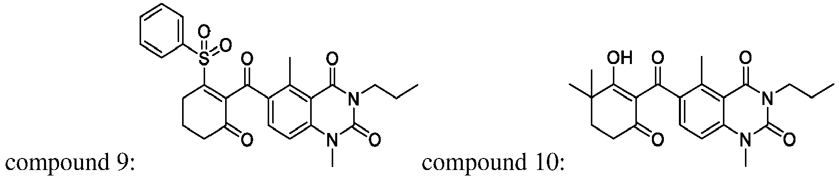

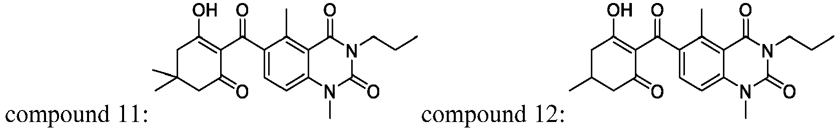

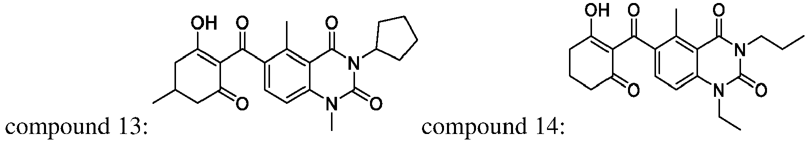

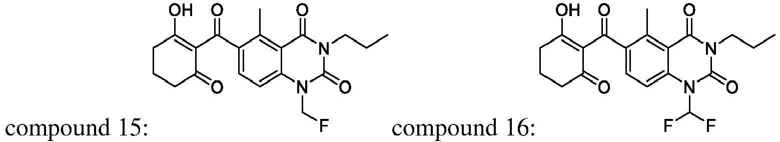

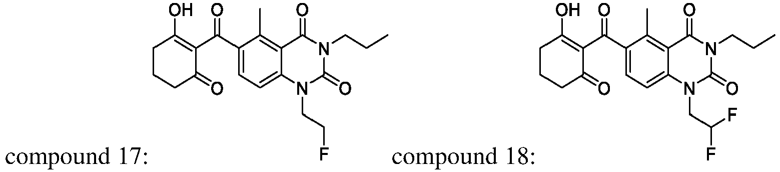

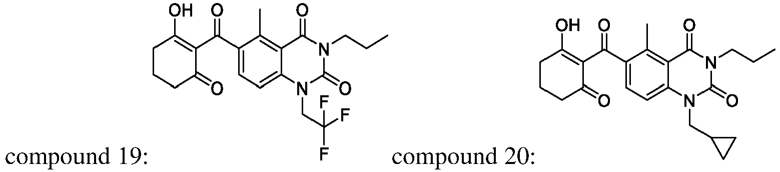

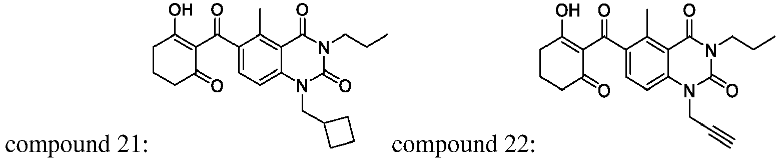

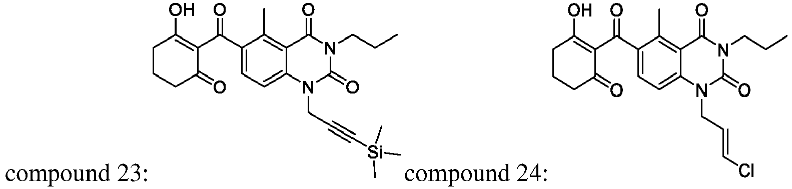

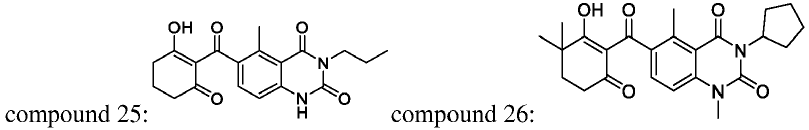

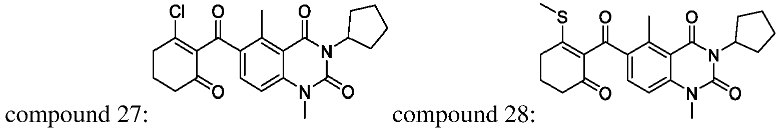

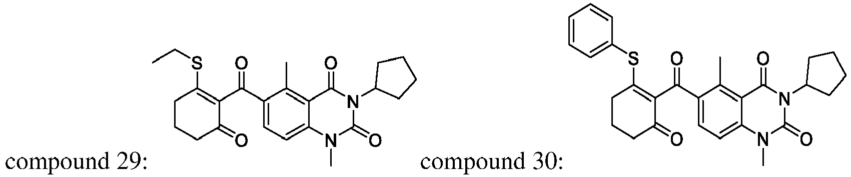

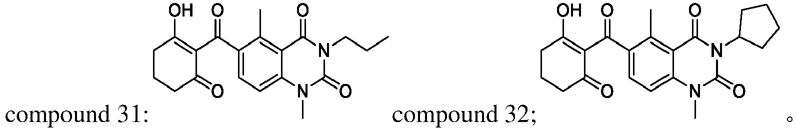

The triketone compounds provided by the invention can be used as HPPD-inhibiting herbicides for controlling weeds in some crop fields such as wheat and/or peanut, and they have good control effect toward multiple gramineous weeds and some broadleaf weeds that are harmful to wheat growth, such as *Echinochloa crusgalli, Digitaria sanguinalis, Setaria viridis, Bromus japonicus, Avena fatua, Aegilops squarrosa,* others. The triketone compounds provided by the invention also have excellent crop safety for wheat and peanut.

The specific method for obtaining the triketone compounds is not particularly limited. Those skilled in the art can obtain suitable methods for preparing the triketone compounds according to the synthesis method in the field of organic synthesis and the specific structure of the triketone compounds provided by the invention.

However, in order to increase the yield of the obtained triketone compounds, as mentioned above, the second aspect of the invention provides a method for preparing the aforementioned triketone compounds, which comprises:
(1) Reacting the compounds with the structure shown in the formula (II-1) with sulfoxide chloride and the compounds with the structure shown in the formula (II-2) in sequence to obtain the compounds with the structure shown in the formula (II-3);
(2) Under the rearrangement reaction condition, the compounds with the structure shown in the formula (II-3) are contacted with the catalysts in the presence of alkalis and solvents to obtain the compounds with the structure shown in the formula (II-4);
   optionally, the method further comprises 1, 2, or 3 of the following steps in sequence:
   (a) Carrying out halogenation reaction on the compounds with the structure shown in the formula (II-4) to obtain the compounds with the structure shown in the formula (II-5);
   (b) Reacting the compounds with the structure shown in the formula (II-5) with sodium mercaptides with the structure shown in the formula (II-6) to obtain the compounds with the structure shown in the formula (II-7);
   (c) Carrying out oxidation reaction on the compounds with the structure shown in the formula (II-7) in the presence of peroxide with the structure shown in the formula (II-8) to obtain the compounds with the structure shown in the formula (II-9); 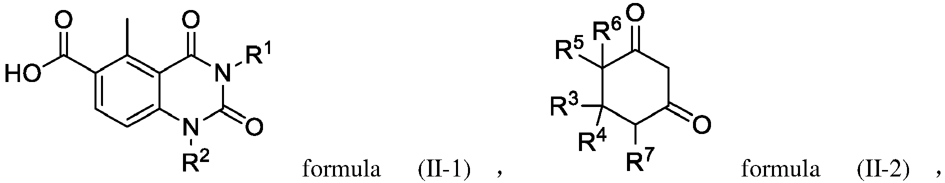 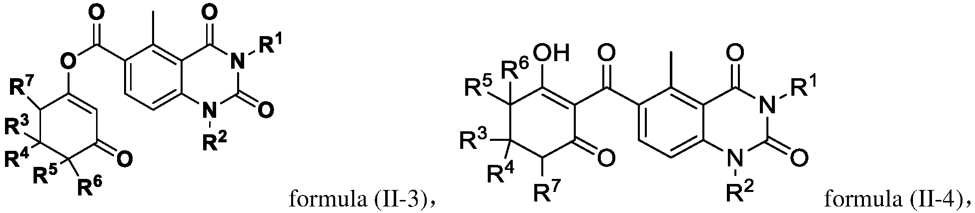 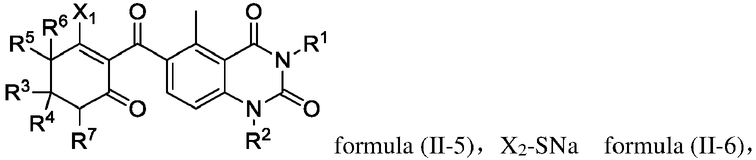 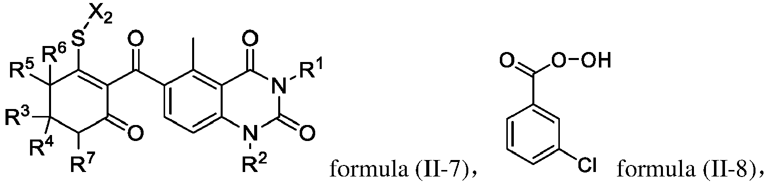 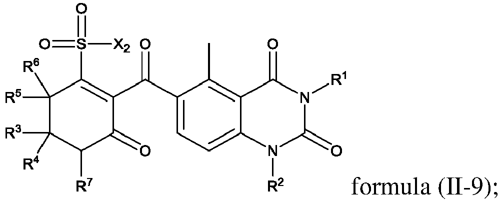
wherein,
X₁ in the compounds with the structure shown in the formula (II-5) is halogen;
X2 in the compounds with the structure shown in the formula (II-6), the formula (II-7) and the formula (II-9) is selected from C₁₋₃ alkyl and phenyl;
moreover, the remaining substituents involved in the formula (II-1), the formula (II-2), the formula (II-3), the formula (II-4), the formula (II-5), the formula (II-7), and the formula (II-9) are the same as those of the aforementioned compounds of the invention.

According to the method for preparing the triketone compounds with the structure shown in the formula (I) in the invention, those skilled in the art can contact the compound with the structure shown in the formula (II-3) and the catalyst in the presence of alkali and solvent according to the conventional conditions and operations of rearrangement reaction.

Preferably, the molar ratio of the compounds having the structure shown in the formula (II-3) to the catalysts and the alkalis is 1: (0.01-1): (0.5-4); more preferably, the molar ratio of the compound having the structure shown in the formula (II-3) to the catalysts and the alkalis is 1: (0.05-1): (1-3).

Preferably, in step (2), the aforementioned contracting conditions include: the reaction temperature is 0-100°C, the reaction time is 0.5-36 h; more preferably, in step (2), the aforementioned contracting conditions include: the reaction temperature is 20-40°C, the reaction time is 5-30 h.

Those skilled in the art should understand that the method of the invention may also include the steps of purifying the obtained product. There is no special requirement for the purification method, and various purification methods conventionally used by those skilled in the art can be used. For example, it can be extracted with extracting agents, dried with desiccants, and removed impurities by column chromatography and the like.

In the preparation method of the invention, the compounds with the structure shown in the formula (II-1) can be prepared by conventional reactions in the art.

Illustratively, the compounds having the structure shown in the formula (II-1) can be prepared by the following synthetic route:

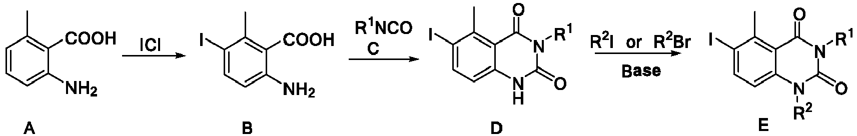

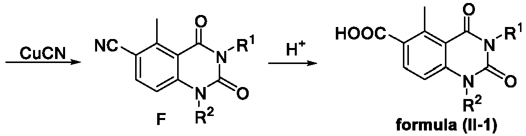

Specifically, the compounds shown in formula A react with iodine chloride to obtain the compounds shown in formula B. Furthermore, the compounds shown in the formula B react with the compounds shown in the formula C to obtain the compounds shown in the formula D, and then the compounds shown in the formula D react with halides (R²I or R²Br) in the presence of alkalis to obtain the compounds shown in the formula E. The compounds shown in the formula E react with cuprous cyanide to obtain the compounds shown in the formula F, and further hydrolysis is carried out under acidic conditions to obtain the compounds shown in the formula (II-1).

In the above synthetic routes, unless otherwise specified, the definitions of the substituents mentioned are the same as the corresponding definitions hereinbefore.

The examples section of this invention exemplify the specific operating conditions in the above synthetic route, and those skilled in the art should not understand as limiting the invention.

Preferably, in step (2), the catalyst is one at least selected from sodium cyanide, potassium cyanide, acetone cyanohydrin, trimethylsilyl cyanide, 1,2, 4-triazole, and 1,2,4-benzotriazole.

Preferably, in step (2), the alkalis is one at least selected from potassium carbonate, sodium carbonate, cesium carbonate, triethylamine, and pyridine.

Preferably, in step (2), the solvents are one at least selected from dichloromethane, trichloromethane, dichloroethane, acetonitrile, toluene, tetrahydrofuran, and benzene.

As mentioned above, the third aspect of the invention provides the use of the aforementioned triketone compounds for controlling weeds.

The weeds mentioned in the invention are those plants that grow in sites where are harmful to human survival and activities and can be non-cultivated wild plants or plants that are useless to humans. For example, the weeds mentioned in the invention can be a variety of wild plants in the field in crop cultivation fields.

In the aforementioned use of the invention, the aforementioned weeds can be broadleaf weeds and/or gramineous weeds.

Preferably, the aforementioned weeds are those weeds that grow in wheat fields and/or peanut fields.

According to a preferred embodiment, the aforementioned weeds are one at least of *Echinochloa crusgalli, Setaria viridis, Digitaria sanguinalis, Amaranthus tricolor, Chenopodium album, Abutilon theophrasti, Aegilops squarrosa, Avena fatua, Bromus japonicus, Alopecurus aequalis, Alopecurus japonicus, Sclerochloa dura, Polypogon fugax, Beckmannia syzigachne, Galium aparine, Malachium aquaticum, Veronica didyma, Cardamine hirsuta, Polygonum lapathifolium, Stellaria alsine,* and *Vicia gigantea.*

Preferably, in the aforementioned use of the invention, the triketone compounds are used at the dosage of 10 g/ha to 400 g/ha.

In the use of the aforementioned triketone compounds provided by the invention, the triketone compounds are dissolved and diluted by solvents for use, and the concentration of the triketone compounds dissolved and diluted by the solvents is preferably 0.05-0.4 g/L. The solvents for dissolving the triketone compounds can include *N*, *N*-dimethylformamide, dimethylsulfoxydumand and the like, and the reagents for dilution can be water containing conventional additives and the like. Preferably, additives commonly used in herbicides in the art, such as one or more surfactants, emulsifiers, and the like, can be added to the solution in which the triketone compounds are dissolved. The diluted triketone compounds of the invention can be sprayed to the stem and/or leaves of plants by conventional methods in the art.

As mentioned above, the fourth aspect of the invention provides a herbicide, which comprises active ingredients and adjuvants, wherein, the active ingredients comprise one at least of the aforementioned triketone compounds.

Preferably, the aforementioned active ingredients are present in an amount of 1wt% to 99.9wt%.

The aforementioned adjuvants of the invention are various additives commonly used in the art for preparing various formulations of herbicides.

Preferably, the formulations of the herbicide of the invention are selected one at least from emulsifiable concentrate, suspension concentrate, wettable powder, powder, granule, aqueous solution, mother liquor, and mother powder.

The invention will be described in detail by giving examples. In the following examples, unless otherwise specified, the various raw materials used in these examples are all commercially available, and their purity all at analytical purity levels.

Unless otherwise specified, the room temperature below all means 25°C±3°C.

### Preparation example 1: preparation of compound 31

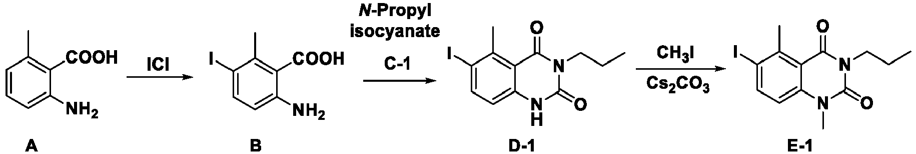

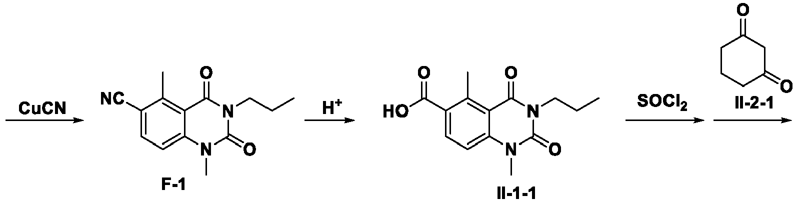

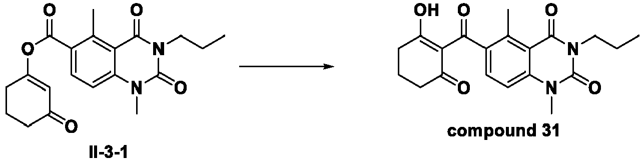

75.5 g of the compounds represented by formula A is added into a 1 L reaction flask at room temperature, and 300 mL of glacial acetic acid is added into the reaction flask with stirring. 81 g of ICl is dissolved in 200 mL of glacial acetic acid, and the solution is added dropwise to the reaction system with stirring within 30 min. After the completion of the dropwise addition, the reaction is continued with stirring for about 2.5 h. After the reaction is finished, the reaction solution is filtered under reduced pressure, and the obtained solids are washed with 200 mL of acetonitrile and 200 mL of glacial acetic acid, respectively, and dried to obtain intermediate B with a yield of 95% and the melting point is 186-188°C. ¹H NMR (600 MHz, DMSO-*d*₆): δ 8.97 (brs, 3H), 7.72 (d, *J*=8.4Hz, 1H), 6.75 (d, *J*=7.8Hz, 1H), 2.40 (s, 3H).

8.31 g of intermediate B is added into a 200 mL two-necked flask, and then 80 mL of pyridine is added. 2.55 g of *N*-propyl isocyanate represented by C-1 is added slowly to the reaction system with stirring. The reaction solution is heated to 100°C and reacted overnight. After the reaction is finished, the pyridine is distilled off under reduced pressure. And the obtained solids are dissolved in acetone, stirred, and passed through the column to obtain intermediate D-1 with a yield of 78% and the melting point is 295-296°C. ¹H NMR (600 MHz, CDCl₃) δ 9.67 (s, 1H), 8.14 (d, *J*=7.8Hz, 1H), 7.40 (d, *J*=7.8Hz, 1H), 3.45 (t, *J*=7.2Hz, 2H), 2.34 (s, 3H), 1.72-1.32 (m, 2H), 0.85 (t, *J*=7.2Hz, 3H).

7.91 g of intermediate D-1 is added into a 200 mL single-necked flask, and then 50 mL of DMF is added. 14.95 g of Cs₂CO₃ is added with stirring and the reaction is continued for about 30 min. 6.49 g of CH₃I is added dropwise slowly to the reaction system, and after the completion of the addition, the reaction is continued with stirring at room temperature overnight. After the reaction is finished, the reaction system is poured into 200 mL of water, and a large number of solids precipitated to obtain intermediate E-1 with a yield of 95% and the melting point is 208-210°C. ¹H NMR (600 MHz, CDCl₃) δ 8.14 (d, *J*=7.8Hz, 1H), 7.40 (d, *J*=7.8Hz, 1H), 3.89 (s, 3H), 3.45 (t, *J*=7.8Hz, 2H), 2.34 (s, 3H), 1.54 (dd, *J*=14.4, 7.8Hz, 2H), 0.85 (t, *J*=6.6Hz, 3H).

7.82 g of intermediate E-1 and 3.89 g of CuCN are added to a 200 mL two-necked flask, and then 100 mL of dried DMF is added. The reaction is refluxed for 12 h, and then DMF is distilled off under reduced pressure. After cooling, 100 mL of acetone is added into the reaction flask and stirred vigorously for 20 mins, and the unreacted CuCN is removed by filtration. The filtrate is dried to obtain intermediate F-1 with a yield of 88% and the melting point is 210-211°C. ¹H NMR (600 MHz, CDCl₃) δ 7.91 (d, *J*=7.8Hz, 1H), 7.36 (d, *J*=7.8Hz, 1H), 3.92 (s, 3H), 3.48 (t, *J*=7.8Hz, 2H), 2.38 (s, 3H), 1.64 (dd, *J*=14.4, 7.8Hz, 2H), 0.95 (t, *J*=6.6Hz, 3H).

4.88 g of intermediate F-1 is added into a 200 mL reaction flask, and then 30 mL of glacial acetic acid, 30 mL of water, and 30 mL of concentrated sulfuric acid are added with stirring. The reaction solution is heated to 120°C and reacted for 12 h. After the reaction is finished, the reaction solution is cooled to room temperature, and then poured into a beaker with 200 mL of ice water. 100 mL of ethyl acetate is added into the beaker to extract and separate the organic layer. The water layer is extracted 2 times by using 100 mL of ethyl acetate and combine the organic layers after the extraction is finished. The organic layer is extracted 3 times by using 30 mL of 50 weight percent of sodium hydroxide solution each time. After the water layers are combined, the water layer is acidified with concentrated hydrochloric acid to the pH = 1, and then stand still to precipitate a large number of solids (namely intermediate II-1-1). The obtained solids are filtered with suction and dried into the next reaction directly.

1.38 g of intermediate II-1-1 is added to a 100 mL single-necked flask, and then 20 mL of dried THF is added. 1.18 g of SOCl₂ is added dropwise slowly to the reaction system at room temperature, the reaction system is refluxed at 75°C for about 1.5 h after the completion of the addition. TLC is used to detect the process of the reaction, and the solvent is removed after the completion of the reaction. 20 mL of dried CHCl₃, 1.12g of the compound represented by formula II-2-1, 1.01 g of Et3N are added into the reaction system to react for about 0.5 h, and TLC is used to track it until the acid chloride disappeared. After the reaction is finished, the reaction system is washed with 100 mL of water once, 30 mL of 1 mol/L HCl for 2 times each time, 30 mL of saturated NaHCO₃ for 2 times each time in sequence, and then dried with anhydrous Na₂SO₄ and passed through a column to obtain intermediate II-3-1 with a yield of 80% and the melting point is 177-178°C. ¹H NMR (600 MHz, CDCl₃) δ 8.25 (d, *J*=9.0Hz, 1H), 7.82 (d, *J*=9.0Hz, 1H), 5.87 (t, *J*=1.8Hz, 1H), 3.89 (s, 3H), 3.45 (t, *J*=7.2Hz, 2H), 3.16 (t, *J*=7.2Hz, 2H), 2.48 (td, *J*=6.6, 1.8Hz, 2H), 2.34 (s, 3H), 1.74-1.31 (m, 4H), 0.85 (t, *J*=6.6Hz, 3H).

1.48 g of intermediate II-3-1 is added into a 50 mL two-necked flask, and then 20 mL of anhydrous acetonitrile is added. 0.81 g of Et3N and 34 mg of acetone cyanohydrin are added under the protection of N2. The reaction system is reacted at room temperature for 8 h, and tracked by TLC until the starting material disappeared. After the reaction is finished, the acetonitrile is stripped off and about 30 mL of CHCl₃ is added. The organic layer is washed with 10 mL of 1 mol/L HCl for 3 times each time, 10 mL of saturated sodium chloride for 3 times each time, and dried over anhydrous sodium sulfate. The solvent is removed under reduced pressure to obtain the pale yellow oil, which is recrystallized with20 mL of methanol to obtain compound 31 (white solid).

### Preparation example 2: preparation of compound 2

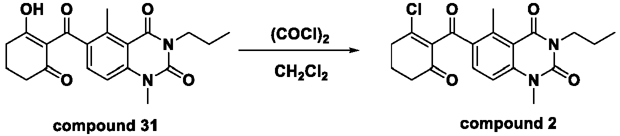

0.925 g of compound 31 is added into a 50 mL two-necked flask, and then 15 mL of anhydrous dichloromethane is added. 0.945 g of oxalyl chloride is added slowly to the reaction system. The reaction system is reacted at room temperature for 15 h and tracked by TLC until the starting material disappeared. After the reaction is finished, the organic layer is washed with saturated sodium bicarbonate for 3 times, and the organic layers are combined and dried over anhydrous sodium sulfate. The solvent is removed under reduced pressure to obtain the white solid, namely the compound 2.

### Preparation example 3: preparation of compound 3

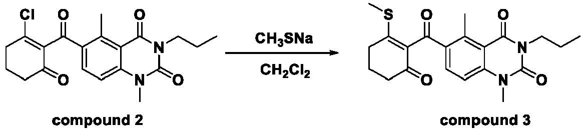

0.31 g of compound 2 is added to a 50 mL two-necked flask, and then 15 mL of anhydrous dichloromethane and 0.112 g of sodium thiomethoxide are added. The reaction system is reacted at room temperature for 30 h, and tracked by TLC until the starting material disappeared. After the reaction is finished, the sample is mixed directly and column chromatography is used to obtain compound 3.

### Preparation example 4: preparation of compound 7

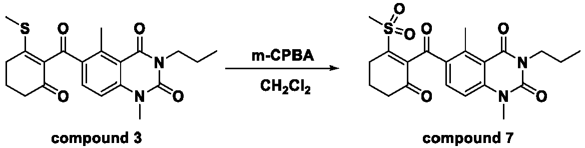

0.2 g of compound 3 is added to a 50 mL two-necked flask, and then 15 mL of anhydrous dichloromethane and 0.171 g of m-chloroperoxybenzoic acid (m-CPBA) are added. The reaction system is reacted at room temperature for 10 h, and tracked by TLC until the starting material disappeared. After the reaction is finished, the sample is mixed directly and column chromatography is used to obtain compound 7.

### Preparation example 5: preparation of compound 32

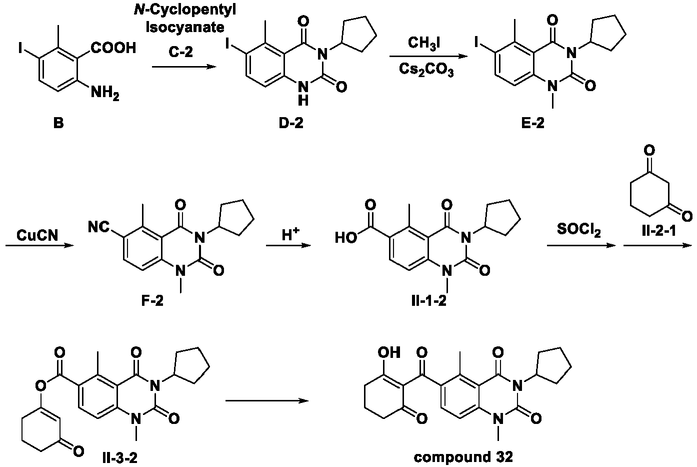

8.31 g of intermediate B is charged into a 200 mL two-necked flask, and then 80 mL of pyridine is added. 3.33 g of cyclopentyl isocyanate represented by C-2 is added slowly to the reaction system with stirring. The reaction solution is heated to 100°C and reacted overnight. After the reaction is finished, the pyridine is distilled off under reduced pressure, and the obtained solids are dissolved in acetone, stirred, and passed through the column to obtain intermediate D-2 with a yield of 80% and the melting point is 307-308°C. ¹H NMR (600 MHz, CDCl₃) δ 9.63 (s, 1H), 8.09 (d, *J*=8.4Hz, 1H), 7.36 (d, *J*=8.4Hz, 1H), 5.96-5.71 (m, 1H), 2.33 (s, 3H), 2.06-1.83 (m, 2H), 1.81-1.50 (m, 6H).

8.88 g of intermediate D-2 is added into a 200 mL single-necked flask, and then 50 mL of DMF is added. 15.6 g of Cs₂CO₃ is added with stirring and the reaction is continued for about 30 min. 6.77 g of CH₃I is added dropwise slowly to the reaction system. After the completion of the addition, the reaction is continued with stirring overnight at room temperature. After the reaction is finished, the reaction system is poured into 200 mL of water, and a large amount of solids is precipitated to obtain intermediate E-2 with a yield of 95% and the melting point is 220-221°C. ¹H NMR (600 MHz, CDCl₃) δ 8.12 (d, *J*=7.8Hz, 1H), 7.38 (d, *J*=7.8Hz, 1H), 4.12-4.08 (m, 1H), 3.89 (s, 3H), 2.34 (s, 3H), 2.09-1.83 (m, 2H), 1.87-1.43 (m, 6H).

8.755 g of intermediate E-2 and 4.06 g of CuCN are added into a 200 mL two-necked flask, and then 100 mL of dried DMF is added. The reaction is refluxed for 12 h, and then DMF is distilled off under reduced pressure. After cooling, 100 mL of acetone is added into the reaction flask and stirred vigorously for 20 mins, and the unreacted CuCN is removed by filtration. The filtrate is dried to obtain intermediate F-2 with a yield of 86% and the melting point is 232-233°C. ¹H NMR (600 MHz, CDCl₃) δ 7.92 (d, *J*=7.8Hz, 1H), 7.37 (d, *J*=7.8Hz, 1H), 4.20-4.12 (m, 1H), 3.92 (s, 3H), 2.42 (s, 3H), 2.15-1.99 (m, 2H), 1.90-1.46 (m, 6H).

5.54 g of intermediate F-2 is added into a 200 mL reaction flask, and then 30 mL of glacial acetic acid, 30 mL of water, and 30mL of concentrated sulfuric acid are added with stirring. The reaction solution is heated to 120°C and reacted for 12 h. After the reaction is finished, the reaction solution is cooled to room temperature, and then poured into a beaker with 200 mL of ice water. 100 mL of ethyl acetate is added into the beaker to extract and separate the organic layer. The water layer is extracted 2 times by using 100 mL of ethyl acetate and combine the organic layers after the extraction is finished. The organic layer is extracted 3 times by using 30 mL of 50 weight percent of sodium hydroxide solution each time. After the water layers are combined, the water layer is acidified with concentrated hydrochloric acid to pH = 1, and then stand still to precipitate a large number of solids (namely intermediate II-1-2). The obtained solids are filtered with suction and dried into the next reaction directly.

1.51 g of intermediate II-1-2 is added to a 100 mL single-necked flask, and then 25 mL of dried THF is added. 1.18 g of SOCl₂ is added dropwise slowly to the reaction system at room temperature, the reaction system is refluxed at 75°C for about 1.5 h after the completion of the addition. TLC is used to track the progress of the reaction, and the solvent is removed after the completion of the reaction. 20 mL of dried CHCl₃, 1.12 g of the compounds represented by formula II-2-1, 1.01 g of Et3N are added into the reaction system to react for about 0.5 h, and TLC is used to track it until the acid chloride disappeared. After the reaction is finished, the reaction system is washed with 100 mL of water once, 30 mL of 1 mol/L HCl for 2 times each time, 30 mL of saturated NaHCO₃ for 2 times each time in sequence, and then dried with anhydrous Na₂SO₄ and passed through a column to obtain intermediate II-3-2 with a yield of 70% and the melting point is 180-181°C. ¹H NMR (600MHz, CDCl₃) δ 8.25 (d, *J*=8.4Hz, 1H), 7.82 (d, *J*=8.4Hz, 1H), 5.85 (s, 1H), 4.31-4.05 (m, 1H), 3.89 (s, 3H), 3.16 (t, *J*=7.8Hz, 2H), 2.51 (td, *J*=6.6, 1.8Hz, 2H), 2.34 (s, 3H), 2.02-1.87 (m, 2H), 1.83-1.60 (m, 6H), 1.54 (p, *J*=6.6Hz, 2H).

1.38 g of intermediate II-3-2 is added into a 50 mL two-necked flask, and then 25 mL of anhydrous acetonitrile is added. 0.71 g of Et3N and 30 mg of acetone cyanohydrin are added under the protection of N2. The reaction system is reacted at room temperature for 10 h, and tracked by TLC until the starting material disappeared. After the reaction is finished, the acetonitrile is stripped off and about 30 mL of CHCl₃ is added. The organic layer is washed with 10 mL of 1 mol/L HCl for 3 times each time, 10 mL of saturated sodium chloride for 3 times each time, and dried over anhydrous sodium sulfate. The solvent is removed under reduced pressure to obtain the pale yellow oil, which is recrystallized with 20 mL of methanol to obtain compound 32 (white solid).

The preparation methods of the remaining compounds of the invention refer to the preparation examples 1 to 5 mentioned above. The characterization data for some specific compounds in the general structure of the invention are listed in table 1:

**Table 1**

| Compound Number | Yield | Melting Point | Nuclear Magnetism |
|---|---|---|---|
| 1 | 78% | 173-174°C | ¹H NMR (400 MHz, CDCl₃) δ 17.67 (s, 1H), 7.31 (d, *J* = 8.8 Hz, 1H), 7.08 (d, *J* = 8.8 Hz, 1H), 5.41 (p, *J* = 8.8 Hz, 1H), 3.56 (s, 3H), 2.67 (s, 3H), 2.66 (s, 2H), 2.30 (s, 2H), 2.20 - 2.08 (m, 2H), 2.05 - 1.93 (m, 2H), 1.90 - 1.81 (m, 2H), 1.67 - 1.56 (m, 2H), 1.12 (s, 6H). ¹³C NMR (125 MHz, CDCl₃) δ 198.8, 197.7, 193.4, 162.5, 150.6, 142.3, 139.7, 135.6, 131.2, 114.3, 113.3, 111.1, 54.1, 52.0, 46.7, 31.24, 31.17, 30.8, 28.33, 28.27, 25.8, 19.72, 19.70. |
| 2 | 94% | 165-166°C | ¹H NMR (400 MHz, CDCl₃) δ 7.69 (d, *J* = 8.8 Hz, 1H), 7.07 (d, *J* = 8.8 Hz, 1H), 4.06 - 3.96 (m, 2H), 3.59 (s, 3H), 3.01 (s, 3H), 2.86 (t, *J* = 6.0 Hz, 2H), 2.55 (t, *J* = 6.8 Hz, 2H), 2.18 (p, *J* = 6.8 Hz, 2H), 1.69 (h, *J* = 7.6 Hz, 2H), 0.97 (t, *J* = 7.6 Hz, 3H). ¹³C NMR (125 MHz, CDCl₃) δ 194.4, 193.3, 161.6, 155.0, 150.6, 146.1, 144.0, 139.9, 135.0, 133.5, 115.1, 111.1, 43.7, 36.8, 34.6, 31.5, 21.7, 21.0, 19.0, 11.4. |
| 3 | 87% | 126-127°C | ¹H NMR (400 MHz, CDCl₃) δ 7.55 (d, *J* = 8.8 Hz, 1H), 7.05 (d, *J* = 8.8 Hz, 1H), 4.05 - 3.95 (m, 2H), 3.57 (s, 3H), 2.86 (s, 3H), 2.83 (t, *J* = 6.0 Hz, 2H), 2.46 - 2.42 (m, 2H), 2.42 (s, 3H), 2.13 (p, *J* = 6.8 Hz, 2H), 1.72 - 1.65 (m, 2H), 0.97 (t, *J* = 7.2 Hz, 3H). ¹³C NMR (125 MHz, CDCl₃) δ 196.2, 193.1, 170.9, 161.9, 150.8, 143.0, 137.0, 134.1, 133.6, 114.5, 111.0, 43.6, 37.1, 31.4, 31.3, 29.9, 21.9, 21.0, 19.2, 15.0, 11.4. |
| 4 | 95% | 82-83°C | ¹H NMR (400 MHz, CDCl₃) δ 7.56 (d, *J* = 8.8 Hz, 1H), 7.05 (d, *J* = 8.8 Hz, 1H), 4.06 - 3.95 (m, 2H), 3.58 (s, 3H), 2.97 - 2.91 (m, 2H), 2.89 (s, 3H), 2.85 (t, *J* = 6.0 Hz, 2H), 2.44 (t, *J* = 6.4 Hz, 2H), 2.13 (p, *J* = 6.0 Hz, 2H), 1.73 - 1.65 (m, 2H), 1.32 (t, *J* = 7.6 Hz, 3H), 0.97 (t, *J* = 7.6 Hz, 3H). ¹³C NMR (125 MHz, CDCl₃) δ 196.3, 193.6, 161.4, 153.7, 150.5, 147.0, 144.0, 135.9, 132.3, 130.9, 114.2, 109.7, 57.4 42.5, 37.9, 31.0, 26.1, 22.4, 21.3, 18.2, 15.8, 11.4. |
| 5 | 85% | 180-181°C | ¹H NMR (400 MHz, CDCl₃) δ 7.58 (d, *J* = 8.8 Hz, 1H), 7.05 (d, *J* = 8.8 Hz, 1H), 4.05 - 3.95 (m, 2H), 3.58 (s, 3H), 2.96 - 2.90 (m, 2H), 2.89 (s, 3H), 2.84 (t, *J* = 6.0 Hz, 2H), 2.46 (t, *J* = 6.4 Hz, 2H), 2.13 (p, *J* = 6.0 Hz, 2H), 1.79 - 1.90 (m, 2H), 1.73 - 1.65 (m, 2H), 1.30 (t, *J* = 7.6 Hz, 3H), 0.97 (t, *J* = 7.6 Hz, 3H). ¹³C NMR (125 MHz, CDCl₃) δ 196.8, 193.9, 161.6, 153.3, 150.6, 147.1, 144.2, 144.1, 136.5, 132.2, 115.2, 110.8, 57.1, 43.7, 37.6, 31.4, 26.1, 21.8, 21.0, 18.8, 15.3, 13.1, 11.3. |
| 6 | 89% | 190-191°C | ¹H NMR (400 MHz, CDCl₃) δ 7.64 (d, *J* = 8.8 Hz, 1H), 7.51 (d, *J* = 8.0 Hz, 2H), 7.47 (d, *J* = 6.8 Hz, 1H), 7.45 - 7.39 (m, 2H), 7.10 (d, *J* = 8.8 Hz, 1H), 4.05 - 3.96 (m, 2H), 3.60 (s, 3H), 2.92 (s, 3H), 2.44 - 2.37 (m, 4H), 1.95 (p, *J* = 6.0 Hz, 2H), 1.69 (h, *J* = 7.2 Hz, 2H), 0.97 (t, *J* = 7.2 Hz, 3H). ¹³C NMR (125 MHz, CDCl₃) δ 196.1, 193.7, 169.6, 161.9, 150.7, 143.3, 143.1, 136.7, 135.5, 134.5, 133.8, 130.3, 129.7, 129.2, 114.6, 111.1, 43.6, 37.4, 31.4, 30.9, 22.1,21.0, 19.3, 11.4. |
| 7 | 90% | 162-163°C | ¹H NMR (400 MHz, CDCl₃) δ 7.76 (d, *J* = 8.8 Hz, 1H), 7.08 (d, *J* = 8.8 Hz, 1H), 4.06 - 4.02 (m, 2H), 3.60 (s, 3H), 3.14 (s, 3H), 3.12 (s, 3H), 2.93 (t, *J* = 5.6 Hz, 2H), 2.64 - 2.58 (m, 2H), 2.28 (p, *J* = 6.0 Hz, 2H), 1.74 - 1.69 (m, 2H), 1.01 (t, *J* = 7.2 Hz, 3H). ¹³C NMR (125 MHz, CDCl₃) δ 196.7, 194.0, 161.6, 153.7, 150.6, 147.2, 144.2, 143.9, 136.5, 132.1, 115.3, 110.9, 43.7, 43.3, 37.5, 31.4, 25.6, 21.6, 21.0, 17.1, 11.4. |
| 8 | 92% | 165-166°C | ¹H NMR (400 MHz, CDCl₃) δ 7.74 (d, *J* = 8.8 Hz, 1H), 7.06 (d, *J* = 8.8 Hz, 1H), 4.06 - 3.97 (m, 2H), 3.57 (s, 3H), 3.28 - 3.17 (m, 2H), 3.09 (s, 3H), 2.88 (t, *J* = 6.0 Hz, 2H), 2.59 (t, *J* = 6.8 Hz, 2H), 2.24 (p, *J* = 6.4 Hz, 2H), 1.69 (h, *J* = 7.6 Hz, 2H), 1.40 (t, *J* = 7.6 Hz, 3H), 0.96 (t, *J* = 7.6 Hz, 3H). ¹³C NMR (125 MHz, CDCl₃) δ 196.7, 193.9, 161.6, 152.9, 150.6, 147.1, 144.5, 144.1, 136.5, 132.2, 115.2, 110.8, 49.9, 43.7, 37.6, 31.5, 26.3, 21.8, 21.0, 18.8, 11.3, 6.0. |
| 9 | 77% | 159-160°C | ¹H NMR (400 MHz, CDCl₃) δ 7.98 (d, *J* = 7.2 Hz, 2H), 7.80 (d, *J* = 8.8 Hz, 1H), 7.72 (t, *J* = 7.6 Hz, 1H), 7.63 (t, *J* = 7.6 Hz, 2H), 7.10 (d, *J* = 8.8 Hz, 1H), 4.08 - 3.99 (m, 2H), 3.60 (s, 3H), 3.19 (s, 3H), 2.82 - 2.62 (m, 2H), 2.57 - 2.48 (m, 2H), 2.10 (t, *J* = 6.8 Hz, 2H), 1.70 (h, *J* = 7.6 Hz, 2H), 0.98 (t, *J* = 7.6 Hz, 3H). ¹³C NMR (125 MHz, CDCl₃) δ 197.0, 193.5, 161.7, 153.0, 150.7, 147.5, 144.1, 142.2, 137.4, 136.7, 134.8, 132.3, 129.7, 128.9, 115.4, 110.9, 43.7, 37.6, 31.5, 31.4, 25.1, 21.6, 21.0, 19.0, 11.4. |
| 10 | 84% | 152-153°C | ¹H NMR (400 MHz, CDC13) δ 17.66 (s, 1H), 7.30 (d, *J* = 8.8 Hz, 1H), 7.11 (d, *J* = 8.0 Hz, 1H), 4.06 - 3.93 (m, 2H), 3.58 (s, 3H), 2.82 (t, *J* = 6.4 Hz, 1H), 2.67 (s, 3H), 2.47 (t, *J* = 6.4 Hz, 1H), 1.94 - 1.84 (m, 2H), 1.68 (h, *J* = 7.6 Hz, 2H), 1.38 - 1.10 (m, 6H), 0.97 (t, *J* = 7.6 Hz, 3H). ¹³C NMR (125 MHz, CDCl₃) δ 200.3, 198.8, 196.7, 162.0, 150.8, 142.3, 139.4, 136.4, 131.1, 113.9, 112.8, 111.3, 43.5, 41.2, 38.8, 32.4, 31.3, 25.6, 24.5, 21.0, 19.6, 11.4. |
| 11 | 83% | 160-161°C | ¹H NMR (400 MHz, CDCl₃) δ 17.66 (s, 1H), 7.32 (d, *J* = 8.8 Hz, 1H), 7.10 (d, *J* = 8.8 Hz, 1H), 4.00 (t, *J* = 8.0 Hz, 2H), 3.59 (s, 3H), 2.69 (s, 3H), 2.66 (s, 2H), 2.30 (s, 2H), 1.76 - 1.62 (m, 2H), 1.12 (s, 6H), 0.97 (t, *J* = 7.6 Hz, 3H). ¹³C NMR (125 MHz, CDCl₃) δ 198.7, 197.7, 193.4, 161.9, 150.8, 142.4, 139.9, 135.8, 131.3, 114.0, 113.2, 111.2, 52.0, 46.7, 43.5, 31.34, 31.30, 30.9, 28.3, 21.0, 19.6, 11.4. |
| 12 | 88% | 140-141°C | ¹H NMR (400 MHz, CDCl₃) δ17.57 (s, 1H), 7.25 (d, *J* = 8.8 Hz, 1H), 7.03 (d, *J* = 8.8 Hz, 1H), 4.02 - 3.86 (m, 2H), 3.52 (s, 3H), 2.77 (d, *J* = 17.2 Hz, 1H), 2.63 (s, 3H), 2.51 - 2.34 (m, 2H), 2.30 - 2.19 (m, 1H), 2.16 - 2.03 (m, 1H), 1.62 (h, *J* = 7.2 Hz, 2H), 1.06 (d, *J* = 6.4 Hz, 3H), 0.91 (t, *J* = 7.6 Hz, 3H). ¹³C NMR (125 MHz, CDCl₃) δ 199.2, 198.0, 193.5, 161.9, 150.8, 142.4, 139.9, 135.9, 131.3, 114.0, 113.7, 111.2, 46.4, 43.5, 40.9, 31.3, 26.5, 21.0, 20.8, 19.7, 11.4. |
| 13 | 79% | 177-178°C | ¹H NMR (400 MHz, CDCl₃) δ 17.64 (s, 1H), 7.30 (d, *J* = 8.8 Hz, 1H), 7.07 (d, *J* = 8.8 Hz, 1H), 5.41 (p, *J* = 8.8 Hz, 1H), 3.56 (s, 3H), 2.90 - 2.75 (m, 1H), 2.67 (s, 3H), 2.58 - 2.42 (m, 2H), 2.29 - 2.09 (m, 4H), 2.05 - 1.94 (m, 2H), 1.91 - 1.80 (m, 2H), 1.67 - 1.53 (m, 2H), 1.12 (d, *J* = 6.4 Hz, 3H). ¹³C NMR (125 MHz, CDCl₃) δ 199.3, 198.0, 193.5, 162.5, 150.6, 142.3, 139.7, 135.8, 131.1, 114.4, 113.8, 111.0, 54.1, 46.4, 40.9, 31.20, 31.17, 28.3, 26.5, 25.8, 20.8, 19.7. |
| 14 | 79% | 119-120°C | ¹H NMR (400 MHz, CDCl₃) δ 17.68 (s, 1H), 7.31 (d, *J* = 8.8 Hz, 1H), 7.10 (d, *J* = 8.8 Hz, 1H), 4.18 (q, *J* = 7.2 Hz, 2H), 4.04 - 3.94 (m, 2H), 2.80 (t, *J* = 6.4 Hz, 2H), 2.69 (s, 3H), 2.44 (t, *J* = 6.4 Hz, 2H), 2.05 (p, *J* = 6.4 Hz, 2H), 1.69 (h, *J* = 7.6 Hz, 2H), 1.33 (t, *J* = 7.2 Hz, 3H), 0.97 (t, *J* = 7.2 Hz, 3H). ¹³C NMR (125 MHz, CDCl₃) δ 199.4, 198.6, 193.6, 161.9, 150.3, 141.5, 140.1, 135.8, 131.3, 114.2, 111.0, 43.4, 39.3, 38.2, 33.1, 21.0, 19.8, 19.0, 12.5, 11.4. |
| 15 | 81% | 124-125°C | ¹H NMR (400 MHz, CDCl₃) δ 17.59 (s, 1H), 7.34 (d, *J* = 8.8 Hz, 1H), 7.23 (d, *J* = 8.8 Hz, 1H), 6.20 (d, *J* = 52.4 Hz, 2H), 4.00 (t, *J* = 7.6 Hz, 2H), 2.81 (t, *J* = 6.4 Hz, 2H), 2.68 (s, 3H), 2.43 (t, *J* = 6.4 Hz, 2H), 2.06 (p, *J* = 6.4 Hz, 2H), 1.70 (h, *J* = 7.6 Hz, 2H), 0.98 (t, *J* = 7.6 Hz, 3H). ¹³C NMR (125 MHz, CDCl₃) δ 199.2, 198.6, 193.6, 161.5, 150.2, 140.5, 139.9, 137.5, 131.4, 114.21, 114.17, 111.5, 83.3, 81.7, 43.6, 38.1, 33.0, 21.0, 19.62, 19.60, 19.0, 11.4. |
| 16 | 73% | 162-163°C | ¹H NMR (400 MHz, CDCl₃) δ 17.57 (s, 1H), 7.94 (t, *J* = 58.4 Hz, 1H), 7.60 (d, *J* = 8.8, 1H), 7.30 (d, *J* = 8.8 Hz, 1H), 4.03 - 3.93 (m, 2H), 2.81 (t, *J* = 6.4 Hz, 2H), 2.67 (s, 3H), 2.47 - 2.41 (m, 2H), 2.06 (p, *J* = 6.4 Hz, 2H), 1.70 (h, *J* = 7.6 Hz, 2H), 0.98 (t, *J* = 7.6 Hz, 3H). ¹³C NMR (125 MHz, CDCl₃) δ 199.1, 198.6, 193.5, 161.0, 149.4, 139.6, 138.1, 136.5, 130.8, 114.5, 114.1, 113.7, 112.7, 110.7, 108.7, 43.6, 38.1, 33.0, 20.9, 19.8, 19.0, 11.3. |
| 17 | 65% | 109-110°C | ¹H NMR (400 MHz, CDCl₃) δ 17.58 (s, 1H), 7.23 (d, *J* = 8.8 Hz, 1H), 7.16 (d, *J* = 8.8 Hz, 1H), 4.71 (dt, *J* = 47.2, 5.2 Hz, 2H), 4.38 (dt, *J* = 23.6, 5.2 Hz, 2H), 3.97 - 3.89 (m, 2H), 2.73 (t, *J* = 6.4 Hz, 2H), 2.63 (s, 3H), 2.42 - 2.32 (m, 2H), 1.99 (p, *J* = 6.4 Hz, 2H), 1.63 (h, *J* = 7.5 Hz, 2H), 0.91 (t, *J* = 7.4 Hz, 3H). ¹³C NMR (125 MHz, CDCl₃) δ 199.3, 198.6, 193.6, 161.8, 150.8, 142.1, 139.9, 136.3, 131.2, 114.2, 114.2, 111.69, 111.66, 82.3, 81.0, 43.5, 38.2, 33.0, 21.0, 19.82, 19.80, 19.0, 11.4. |
| 18 | 78% | 91-92°C | ¹H NMR (400 MHz, CDCl₃) δ 17.61 (s, 1H), 7.31 (d, *J* = 8.8 Hz, 1H), 7.20 (d, *J* = 8.8 Hz, 1H), 6.13 (tt, *J* = 56.0, 4.8 Hz, 1H), 4.47 (td, *J* = 12.8, 4.8 Hz, 2H), 4.08 - 3.91 (m, 2H), 2.80 (t, *J* = 6.4 Hz, 2H), 2.69 (s, 3H), 2.43 (t, *J* = 6.4 Hz, 2H), 2.05 (p, *J* = 6.4 Hz, 2H), 1.70 (h, *J* = 7.6 Hz, 2H), 0.98 (t, *J* = 7.6 Hz, 3H). ¹³C NMR (125 MHz, CDCl₃) δ 199.2, 198.6, 193.6, 161.6, 151.0, 141.6, 140.1, 136.8, 131.2, 115.5, 114.3, 114.2, 113.5, 111.6, 111.4, 46.6, 46.4, 46.1, 43.6, 38.2, 33.0, 21.0, 19.8, 19.0, 11.4. |
| 19 | 69% | 96-97°C | ¹H NMR (400 MHz, CDCl₃) δ 17.52 (s, 1H), 7.26 (d, *J* = 8.8 Hz, 1H), 7.05 (d, *J* = 8.8 Hz, 1H), 4.76 (s, 2H), 4.03 - 3.88 (m, 2H), 2.74 (t, *J* = 6.4 Hz, 2H), 2.63 (s, 3H), 2.37 (t, *J* = 6.4 Hz, 2H), 1.99 (p, *J* = 6.4 Hz, 2H), 1.68 - 1.59 (m, 2H), 0.91 (t, *J* = 7.6 Hz, 3H). ¹³C NMR (125 MHz, CDCl₃) δ 199.1, 198.6, 193.6, 161.3, 150.7, 141.0, 140.3, 137.1, 131.2, 125.0, 122.8, 114.3, 114.2, 111.2, 44.9, 44.6, 43.9, 38.1, 33.0, 20.9, 19.8, 19.0, 11.3.. |
| 20 | 79% | 82-83°C | ¹H NMR (400 MHz, CDCl₃) δ 17.68 (s, 1H), 7.32 (d, *J* = 8.8 Hz, 1H), 7.24 (d, *J* = 8.8 Hz, 1H), 4.06 (d, *J* = 7.2 Hz, 2H), 4.03 - 3.95 (m, 2H), 2.80 (t, *J* = 6.4 Hz, 2H), 2.70 (s, 3H), 2.44 (t, *J* = 6.4 Hz, 2H), 2.05 (p, *J* = 6.4 Hz, 2H), 1.69 (h, *J* = 7.6 Hz, 2H), 1.28 - 1.15 (m, 1H), 0.97 (t, *J* = 7.6 Hz, 3H), 0.57 - 0.49 (m, 4H). ¹³C NMR (125 MHz, CDCl₃) δ 199.4, 198.6, 193.6, 162.0, 151.0, 142.0, 140.0, 135.8, 131.2, 114.3, 114.1, 111.5, 48.0, 43.5, 38.2, 33.1, 21.0, 19.9, 19.0, 11.4, 9.5, 4.1. |
| 21 | 85% | 90-91°C | ¹H NMR (400 MHz, CDCl₃) δ 17.64 (s, 1H), 7.28 (d, *J* = 8.8 Hz, 1H), 7.04 (d, *J* = 8.8 Hz, 1H), 4.21 (d, *J* = 7.2 Hz, 2H), 4.09 - 3.95 (m, 2H), 2.86 - 2.73 (m, 3H), 2.68 (s, 3H), 2.50 - 2.36 (m, 2H), 2.09 - 2.00 (m, 4H), 1.96 - 1.85 (m, 4H), 1.68 (h, *J* = 7.5 Hz, 2H), 0.96 (t, *J* = 7.4 Hz, 3H). ¹³C NMR (125 MHz, CDCl₃) δ 199.4, 198.6, 193.6, 161.9, 151.1, 141.9, 139.9, 135.7, 131.1, 114.2, 114.1, 111.5, 48.4, 43.5, 38.2, 33.9, 33.1, 26.3, 21.0, 19.9, 19.8, 19.0, 18.4, 11.4. |
| 22 | 81% | 79-80°C | ¹H NMR (400 MHz, CDCl₃) δ 17.65 (s, 1H), 7.35 (d, *J* = 8.8 Hz, 1H), 7.27 (d, *J* = 8.4 Hz, 1H), 4.92 (s, 2H), 4.10 - 3.89 (m, 2H), 2.80 (t, *J* = 6.4 Hz, 2H), 2.69 (s, 3H), 2.44 (t, *J* = 6.4 Hz, 2H), 2.29 (t, *J* = 2.4 Hz, 1H), 2.06 (p, *J* = 6.4 Hz, 2H), 1.77 - 1.63 (m, 2H), 0.98 (t, *J* = 7.6 Hz, 3H). ¹³C NMR (125 MHz, CDCl₃) δ 199.3, 198.6, 193.6, 161.7, 150.2, 140.8, 140.0, 136.5, 131.2, 114.2, 111.7, 73.30, 73.28, 43.7, 43.6, 38.2, 33.8, 33.0, 21.0, 19.7, 19.0, 11.4. |
| 23 | 77% | 127-128°C | ¹H NMR (400 MHz, CDCl₃) δ 17.65 (s, 1H), 7.35 (d, *J* = 8.8 Hz, 1H), 7.29 (d, *J* = 8.8 Hz, 1H), 4.92 (s, 2H), 4.06 - 3.96 (m, 2H), 2.80 (t, *J* = 6.4 Hz, 2H), 2.69 (s, 3H), 2.44 (t, *J* = 6.4 Hz, 2H), 2.06 (p, *J* = 6.4 Hz, 2H), 1.78 - 1.63 (m, 2H), 0.98 (t, *J* = 7.6 Hz, 3H), 0.13 (s, 9H). ¹³C NMR (125 MHz, CDCl₃) δ 199.4, 198.4, 193.5, 161.8, 150.1, 141.1, 139.9, 136.3, 131.1, 114.3, 114.2, 112.0, 98.6, 90.4, 43.7, 38.2, 34.9, 33.0, 21.0, 19.74, 19.72, 19.0, 11.4, 0.08, 0.06 |
| 24 | 69% | 87.3-88.5°C | ¹H NMR (400 MHz, CDCl₃) δ 17.64 (s, 1H), 7.30 (d, *J* = 8.8 Hz, 1H), 7.03 (d, *J* = 8.8 Hz, 1H), 6.29 (dt, *J* = 13.6, 1.6 Hz, 1H), 6.05 (dt, *J* = 13.6, 6.0 Hz, 1H), 4.74 (d, *J* = 6.0 Hz, 2H), 4.06 - 3.93 (m, 2H), 2.80 (t, *J* = 6.4 Hz, 2H), 2.68 (s, 3H), 2.43 (t, *J* = 6.4 Hz, 2H), 2.05 (p, *J* = 6.4 Hz, 2H), 1.74 - 1.62 (m, 2H), 0.97 (t, *J* = 7.6 Hz, 3H). ¹³C NMR (125 MHz, CDCl₃) δ 199.3, 198.6, 193.6, 161.7, 150.4, 141.2, 140.2, 136.4, 131.3, 126.6, 122.8, 114.2, 111.1, 44.0, 43.6, 38.2, 33.0, 21.0, 19.8, 19.0, 11.4. |
| 25 | 66% | 266-267°C | ¹H NMR (400 MHz, CDCl₃) δ 17.69 (s, 1H), 10.34 (s, 1H), 7.23 (d, *J* = 8.4 Hz, 1H), 6.91 (d, *J* = 8.4 Hz, 1H), 4.06 - 3.70 (m, 2H), 2.80 (t, *J* = 6.4 Hz, 2H), 2.68 (s, 3H), 2.47 (t, *J* = 6.4 Hz, 2H), 2.06 (p, *J* = 6.4 Hz, 2H), 1.70 (h, *J* = 7.6 Hz, 2H), 0.98 (t, *J* = 7.6 Hz, 3H). ¹³C NMR (125 MHz, CDCl₃) δ 199.4, 198.8, 194.1, 162.5, 151.6, 140.7, 139.4, 135.8, 131.6, 114.2, 112.9, 112.8, 42.5, 38.2, 33.1, 30.6, 21.1, 19.0, 11.4. |
| 26 | 85% | 144-145°C | ¹H NMR (400 MHz, CDCl₃) δ 17.83 (s, 1H), 7.29 (d, *J* = 8.8 Hz, 1H), 7.08 (d, *J* = 8.0 Hz, 1H), 5.41 (p, *J* = 8.8 Hz, 1H), 3.56 (s, 3H), 2.82 (t, *J* = 6.8 Hz, 1H), 2.65 (s, 3H), 2.46 (t, *J* = 6.8 Hz, 1H), 2.22 - 2.08 (m, 2H), 2.05 - 1.94 (m, 2H), 1.88 (dt, *J* = 11.2, 6.8 Hz, 4H), 1.67 - 1.57 (m, 2H), 1.38 (s, 2H), 1.11 (s, 4H). ¹³C NMR (125 MHz, CDCl₃) δ 200.3, 198.8, 196.7, 162.5, 150.6, 142.2, 139.3, 136.3, 131.2, 130.9, 112.8, 111.2, 54.0, 41.2, 38.8, 34.7, 33.4, 32.4, 31.2, 29.3, 28.3, 25.8, 25.6, 24.5, 19.7. |
| 27 | 85% | 148-149°C | ¹H NMR (400 MHz, CDCl₃) δ 7.69 (d, *J* = 8.8 Hz, 1H), 7.06 (d, *J* = 8.8 Hz, 1H), 5.42 (p, *J* = 8.8 Hz, 1H), 3.59 (s, 3H), 3.01 (s, 3H), 2.87 (t, *J* = 6.0 Hz, 2H), 2.57 (t, *J* = 6.4 Hz, 2H), 2.24 - 2.19 (m, 2H), 2.17 - 2.10 (m, 2H), 2.07 - 1.96 (m, 2H), 1.94 - 1.84 (m, 2H), 1.69 - 1.59 (m, 2H). ¹³C NMR (125 MHz, CDCl₃) δ.194.4, 193.3, 162.2, 154.9, 150.4, 145.9, 144.0, 140.0, 134.9, 133.3, 115.6, 110.9, 54.3, 36.8, 34.6, 31.32, 31.29, 28.4, 25.8, 21.7, 19.1, 19.0. |
| 28 | 92% | 153-154°C | ¹H NMR (400 MHz, CDCl₃) δ 7.54 (d, *J* = 8.8 Hz, 1H), 7.02 (d, *J* = 8.8 Hz, 1H), 5.40 (p, *J* = 8.8 Hz, 1H), 3.55 (s, 3H), 2.84 (s, 3H), 2.82 (t, *J* = 6.0 Hz, 2H), 2.46 - 2.42 (m, 2H), 2.41 (s, 3H), 2.18 - 2.09 (m, 4H), 2.05 - 1.93 (m, 2H), 1.91 - 1.80 (m, 2H), 1.66 - 1.57 (m, 2H). ¹³C NMR (125 MHz, CDCl₃) δ 196.3, 193.1, 170.5, 162.5, 150.6, 142.9, 136.8, 134.2, 133.5, 115.0, 110.9, 54.1, 37.1, 31.22, 31.19, 29.9, 28.4, 25.8, 21.9, 19.3, 19.3, 14.98, 14.97. |
| 29 | 88% | 80-82°C | ¹H NMR (400 MHz, CDCl₃) δ 7.56 (d, *J* = 8.8 Hz, 1H), 7.02 (d, *J* = 8.8 Hz, 1H), 5.40 (p, *J* = 8.8 Hz, 1H), 3.55 (s, 3H), 2.92 (q, *J* = 7.6 Hz, 2H), 2.87 (s, 3H), 2.84 (t, *J* = 6.0 Hz, 2H), 2.48 - 2.40 (m, 2H), 2.19 - 2.07 (m, 4H), 2.03 - 1.94 (m, 2H), 1.90 - 1.80 (m, 2H), 1.65 - 1.56 (m, 2H), 1.31 (t, *J* = 7.6 Hz, 3H). ¹³C NMR (125 MHz, CDCl₃) δ 196.3, 193.3, 169.0, 162.4, 150.6, 143.4, 143.0, 136.4, 134.8, 133.8, 115.1, 110.8, 54.2, 37.2, 31.24, 31.21, 29.9, 28.4, 25.8, 25.7, 22.0, 19.28, 19.25, 14.2. |
| 30 | 84% | 218-219°C | ¹H NMR (400 MHz, CDCl₃) δ 7.63 (d, *J* = 8.8 Hz, 1H), 7.51 (d, *J* = 6.8 Hz, 2H), 7.47 (d, *J* = 6.8 Hz, 1H), 7.45 - 7.39 (m, 2H), 7.07 (d, *J* = 8.8 Hz, 1H), 5.41 (p, *J* = 8.8 Hz, 1H), 3.57 (s, 3H), 2.91 (s, 3H), 2.45 - 2.35 (m, 4H), 2.19 - 2.12 (m, 2H), 2.05 - 1.91 (m, 4H), 1.92 - 1.81 (m, 2H), 1.67 - 1.59 (m, 2H). ¹³C NMR (125 MHz, CDCl₃) δ 196.2, 193.7, 169.3, 162.4, 150.6, 143.2, 143.1, 136.6, 135.5, 134.6, 133.7, 130.2, 129.7, 129.2, 115.0, 111.0, 54.2, 37.4, 31.4, 31.3, 31.2, 28.4, 25.8, 22.1, 19.40, 19.38. |
| 31 | 77% | 155-156°C | ¹H NMR (400 MHz, CDCl₃) δ 17.65 (s, 1H), 7.32 (d, *J* = 8.8 Hz, 1H), 7.09 (d, *J* = 8.8 Hz, 1H), 4.05 - 3.96 (m, 2H), 3.58 (s, 3H), 2.80 (t, *J* = 6.4 Hz, 2H), 2.70 (s, 3H), 2.43 (t, *J* = 6.4 Hz, 2H), 2.05 (p, *J* = 6.4 Hz, 2H), 1.69 (h, *J* = 7.6 Hz, 2H), 0.97 (t, *J* = 7.2 Hz, 3H).¹³C NMR (100 MHz, CDCl₃) δ 199.4, 198.6, 193.7, 161.9, 150.8, 142.4, 139.8, 135.9, 131.3, 114.2, 114.0, 111.2, 43.5, 38.2, 33.0, 31.3, 21.0, 19.7, 19.0, 11.4. |
| 32 | 78% | 185-186°C | ¹H NMR (400 MHz, CDCl₃) δ 17.66 (s, 1H), 7.31 (d, *J* = 6.0 Hz, 1H), 7.07 (d, *J* = 6.8 Hz, 1H), 5.42 (p, *J* = 8.8 Hz, 1H), 3.57 (s, 3H), 2.80 (t, *J* = 6.4 Hz, 2H), 2.69 (s, 3H), 2.44 (t, *J* = 6.4 Hz, 2H), 2.21 - 2.12 (m, 2H), 2.09 - 1.96 (m, 4H), 1.93 - 1.81 (m, 2H), 1.68 - 1.60 (m, 2H).¹³C NMR (100 MHz, CDCl₃) δ 199.5, 198.6, 193.7, 162.5, 150.6, 142.3, 139.7, 135.9, 131.1, 114.4, 111.1, 54.0, 38.2, 33.1, 31.2, 28.3, 25.8, 19.8, 19.0. |

### Test example 1

This test example is intended to illustrate the herbicidal inhibition rate (%) of some specific compounds among the compounds having the structure represented by formula (I) and the comparative compound D1, wherein, the structural formula of the comparative compound D1 (the comparative compound D1 is a specific compound disclosed in CN104557739A, and the main inventors of CN104557739A are the same as this invention) is:

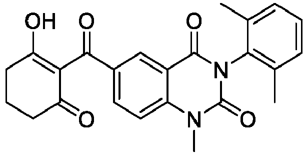

Preliminary screening test (pot-culture method): the targets of the test are *Echinochloa crusgalli, Digitaria sanguinalis, Setaria viridis, Amaranthus tricolor, Chenopodium album, Abutilontheophrasti.* Spraying the stems and leaves after seedlings: taking a paper cup with an inner diameter of 7 cm and fill it with composite soil (vegetable garden soil: seedling substrate, 1: 2, v/v) to 3/4 of the cup. Sowing weeds directly, cover it with 0.2 cm soil and wait until it grows to 4-5 leaves stage for later use. The compounds of the invention and the aforementioned comparative compound D1 are applied in an automatic spray tower at a dose of 150 g.a.i/ha (g/ha). After the liquid on the crop leaves is dried in the air, the crops are transferred to the greenhouse for cultivation (humidity 70%), and the results were investigated after 30 days.

The growth inhibition rate evaluation method is a visual method. The growth inhibition rate is rated specifically according to the conditions shown in table 2, and the test results are shown in Table 3.

**Table 2**

| Growth Inhibition Rate /% | Evaluation (inhibition, malformation, albinism, etc.) | Level |
|---|---|---|
| 0 | has no influence on the growth of weeds or crops and has no drug effect symptom. | Grade 0 (same as blank control) |
| 1-19 | slightly affects the growth of weeds or crops, and has no obvious drug effect symptom. | Level 1 |
| 20-49 | has certain effect on weed or crop growth, and has obvious drug effect. | Level 2 |
| 50-79 | weeds or crops are subject to severe growth inhibition. | Level 3 |
| 80-99 | weeds or crops die. | Level 4 |
| 100 | weeds or crops die completely. | Level 5 |

**Table 3**

| Compound Number | Dosage/g.a.i/ha | Inhibition Level | | | | | |
|---|---|---|---|---|---|---|---|
| | | *Echinochloa crusgalli* | *Setaria viridis* | *Digitaria sanguinalis* | *Amaranthus tricolor* | *Chenopodium album* | *Abutilon theophrasti* |
| 2 | 150 | 5 | 4 | 5 | 3 | 5 | 5 |
| 3 | 150 | 4 | 3 | 4 | 4 | 5 | 5 |
| 4 | 150 | 5 | 4 | 4 | 4 | 4 | 5 |
| 5 | 150 | 5 | 3 | 5 | 5 | 4 | 5 |
| 6 | 150 | 5 | 3 | 5 | 3 | 5 | 5 |
| 7 | 150 | 5 | 3 | 5 | 4 | 5 | 5 |
| 9 | 150 | 5 | 3 | 5 | 4 | 5 | 5 |
| 10 | 150 | 5 | 5 | 5 | 5 | 5 | 5 |
| 11 | 150 | 5 | 5 | 5 | 5 | 5 | 5 |
| 12 | 150 | 5 | 5 | 5 | 5 | 5 | 5 |
| 13 | 150 | 5 | 4 | 3 | 4 | 5 | 5 |
| 14 | 150 | 5 | 5 | 5 | 5 | 5 | 5 |
| 15 | 150 | 5 | 4 | 4 | 4 | 5 | 5 |
| 16 | 150 | 5 | 4 | 5 | 4 | 4 | 5 |
| 17 | 150 | 4 | 3 | 4 | 5 | 4 | 5 |
| 18 | 150 | 5 | 3 | 3 | 5 | 5 | 5 |
| 19 | 150 | 5 | 4 | 5 | 4 | 5 | 5 |
| 20 | 150 | 5 | 3 | 3 | 5 | 5 | 5 |
| 22 | 150 | 4 | 3 | 3 | 5 | 5 | 5 |
| 26 | 150 | 5 | 5 | 4 | 4 | 4 | 5 |
| 31 | 150 | 5 | 5 | 5 | 4 | 5 | 5 |
| 32 | 150 | 5 | 5 | 5 | 5 | 5 | 5 |
| D1 | 150 | 4 | 4 | 4 | 4 | 5 | 4 |

### Test example 2

The same method as in test example 1 is used to conduct re-screen assays at the low dose for representative compounds, and the results are shown in Table 4.

**Table 4**

| Compound Number | Dosage/g.a.i/ha | Inhibition Level | | | | | | | |
|---|---|---|---|---|---|---|---|---|---|
| | | *Echinochloa crusgalli* | *Setaria viridis* | *Digitaria sanguinalis* | *Aegilops squarrosa* | *Avena fatua* | *Bromus japonicus* | *Alopecurus aequalis* | *Alopecurus japonicus* |
| 10 | 120 | 5 | 5 | 5 | 5 | 5 | 5 | 3 | 3 |
| | 60 | 5 | 5 | 4 | 4 | 4 | 3 | 2 | 2 |
| | 30 | 4 | 4 | 4 | 4 | 4 | 2 | 2 | 2 |
| 11 | 120 | 5 | 5 | 5 | 5 | 5 | 4 | 5 | 4 |
| | 60 | 5 | 5 | 5 | 5 | 4 | 4 | 3 | 3 |
| | 30 | 4 | 5 | 4 | 5 | 3 | 3 | 3 | 3 |
| 12 | 120 | 4 | 5 | 5 | 4 | 4 | 3 | 3 | 3 |
| | 60 | 4 | 5 | 4 | 3 | 3 | 3 | 3 | 3 |
| | 30 | 4 | 5 | 4 | 3 | 3 | 2 | 2 | 2 |
| 14 | 120 | 4 | 5 | 5 | 3 | 3 | 4 | 3 | 3 |
| | 60 | 4 | 3 | 3 | 3 | 2 | 3 | 2 | 2 |
| | 30 | 4 | 2 | 2 | 2 | 2 | 2 | 2 | 2 |
| 31 | 120 | 5 | 5 | 5 | 5 | 5 | 5 | 4 | 5 |
| | 60 | 5 | 5 | 5 | 5 | 5 | 4 | 3 | 3 |
| | 30 | 5 | 5 | 5 | 5 | 5 | 3 | 3 | 3 |
| 32 | 120 | 5 | 5 | 5 | 5 | 5 | 5 | 3 | 3 |
| | 60 | 5 | 4 | 5 | 3 | 4 | 3 | 2 | 2 |
| | 30 | 5 | 4 | 3 | 2 | 2 | 2 | 2 | 2 |
| D1 | 120 | 4 | 4 | 4 | 2 | 2 | 0 | 1 | 0 |
| | 60 | 3 | 3 | 3 | 1 | 0 | 0 | 0 | 0 |
| | 30 | 3 | 3 | 3 | 0 | 0 | 0 | 0 | 0 |

From the results shown above, it can be seen that:
In the primary screening of herbicidal activity experiments, most of the compounds of the invention show excellent herbicidal effects against 6 kinds of common gramineous and broadleaf weeds such as *Echinochloa crusgalli, Setaria viridis, Digitaria sanguinalis, Amaranthus tricolor, Chenopodium album,* and *Abutilon theophrasti,* and the activity is equivalent to or obviously better than that of compound D1.

Furthermore, in the re-screening experiments with reduced concentration, the herbicidal activity of the compounds of the invention is still equivalent to or even better than that of the compound D1 toward 3 kinds of weeds such as *Echinochloa crusgalli, Setaria viridis* and *Digitaria sanguinalis.* For the gramineous weeds such as *Aegilops squarrosa, Avenafatua, Bromus japonicus, Alopecurus aequalis,* and *Alopecurus japonicus,* compound 10, 11, 12, 14, 31, and 32 maintain good herbicidal activity on the aforementioned weeds at three application concentrations, while the control compound D1 hardly shows herbicidal activity against the main gramineous weeds in wheat fields.

It is thus found that the compounds of the invention can be used as broad-spectrum herbicides for controlling gramineous weeds.

### Test example 3

The same method as in test example 1 is used to determine crop safety of some compounds, and the results are shown in Table 5.

**Table 5**

| Compound Number | Dosage / g.a.i/ha | Inhibition Level | |
|---|---|---|---|
| | | Wheat | Peanuts |
| 11 | 120 | 1 | 1 |
| | 60 | 1 | 1 |
| | 30 | 1 | 0 |
| 12 | 120 | 2 | 1 |
| | 60 | 2 | 0 |
| | 30 | 1 | 0 |
| 14 | 120 | 2 | 2 |
| | 60 | 1 | 1 |
| | 30 | 1 | 1 |
| 31 | 120 | 1 | 1 |
| | 60 | 1 | 0 |
| | 30 | 0 | 0 |
| 32 | 120 | 0 | 1 |
| | 60 | 0 | 1 |
| | 30 | 0 | 1 |

From the results shown above, it can be seen that:
Compound 11 has only a slight inhibition effect against wheat at three application concentrations, but it does not influence the growth of wheat and has excellent crop safety. By comparison, compounds 12 and 14 have higher safety to wheat at low concentrations.

Compound 31 shows almost no inhibition effect against wheat at three application concentrations, and compound 32 has only a slight inhibition effect against wheat at three application concentrations, but it does not influence the normal growth of wheat.

In combination with the above-mentioned good herbicidal activity toward gramineous weeds which are difficult to be controlled in wheat fields, the compounds of the invention are obviously suitable for being applied as herbicides in wheat fields and have huge commercial value.

In addition, compounds 11, 12, 31 and 32 in the invention are also very safe to peanuts, so they can be used for controlling gramineous weeds and some broadleaf weeds in peanut fields. No HPPD-inhibiting herbicide for controlling the weeds in the peanut fields is available on the market at present.

The preferred embodiments of the invention are described above in detail, but the invention is not limited thereto. Within the scope of the technical ideal of the invention, various simple modifications can be made to the technical solutions of the invention, including various technical features being combined in any other suitable way. These simple modifications and combinations should also be regarded as the disclosure of the invention, and all fall within the scope of the invention.

## Claims

1. A triketone compound has the structure shown in the formula (I), 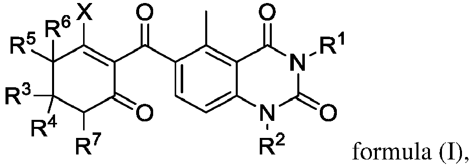 Wherein, in the formula (I),
R¹ is *n*-propyl or cyclopentyl;
R² is selected from H, C₁₋₆ alkyl, C₁₋₃ alkyl substituted by 1 to 6 halogen atoms, C₂₋₆ alkynyl, C₅₋₈ alkynyl substituted by trimethylsilyl, C₂₋₆ alkenyl, C₂₋₆ alkenyl substituted by 1-6 halogen atoms;
X is selected from hydroxyl, halogen, C₁₋₃ alkylthio, phenylthio, C₁₋₃ alkyl sulfuryl and phenyl sulfuryl;
R³, R⁴, R⁵, R⁶, and R⁷ are selected independently from H and C₁₋₃ alkyl.

2. The compounds according to claim 1, wherein, in the formula (I),
R¹ is *n*-propyl or cyclopentyl;
R² is selected from H, C₁₋₃ alkyl, C₁₋₃ alkyl substituted by 1 to 3 halogen atoms, C₃₋₆ alkynyl, C₆₋₈ alkynyl substituted by trimethylsilyl, C₃₋₆ alkenyl, C₃₋₆ alkenyl substituted by 1-3 halogen atoms;
X is selected from hydroxyl, halogen, C₁₋₃ alkylthio, phenylthio, C₁₋₃ alkyl sulfuryl and phenyl sulfuryl;
R³, R⁴, R⁵, and R⁶ are selected independently from H and C₁₋₃ alkyl;
R⁷ is H;
Preferably, in the formula (I),
R¹ is *n*-propyl or cyclopentyl;
R² is selected from H, methyl, ethyl, monofluoromethyl, difluoromethyl, monofluoroethyl, difluoroethyl, trifluoromethyl, trifluoroethyl, 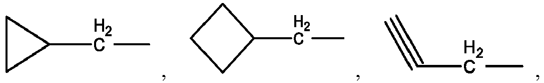 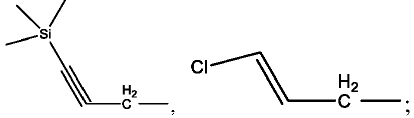 X is selected from-OH, -F, -Cl, -Br, CH₃-S-, CH₃CH₂-S-, CH₃CH₂CH₂-S-, 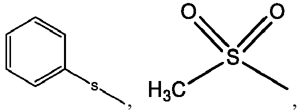 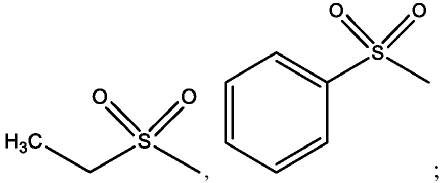 R³, R⁴, R⁵, R⁶ are selected independently from H, methyl, ethyl, n-propyl and isopropyl;
R⁷ is H.

3. The compounds according to claim 1, wherein, the compounds of formula (I) are at least one selected from the followings:

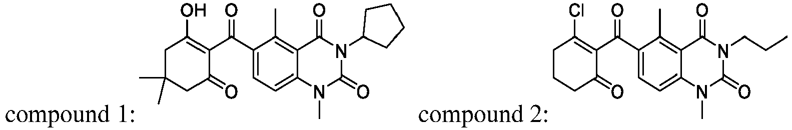

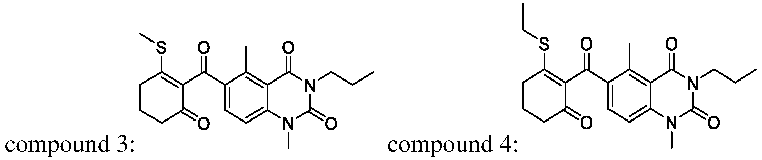

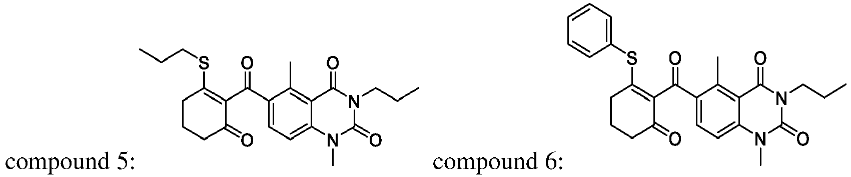

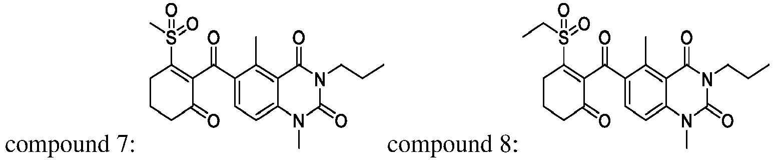

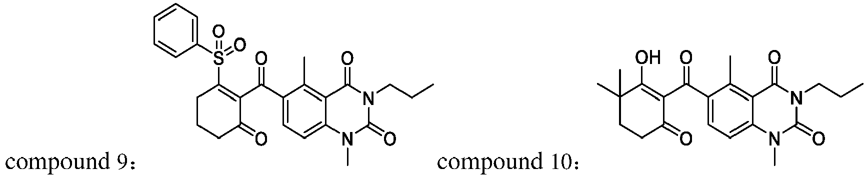

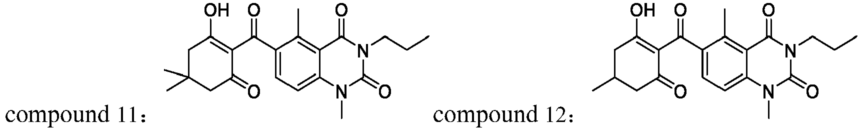

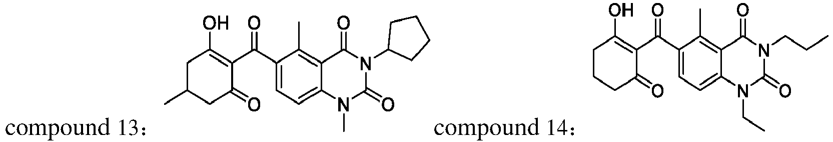

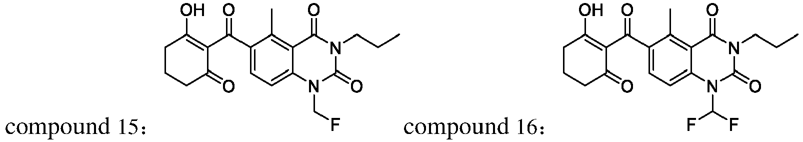

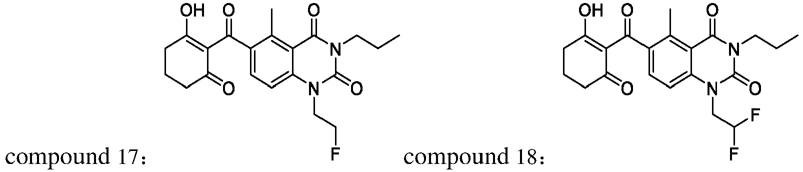

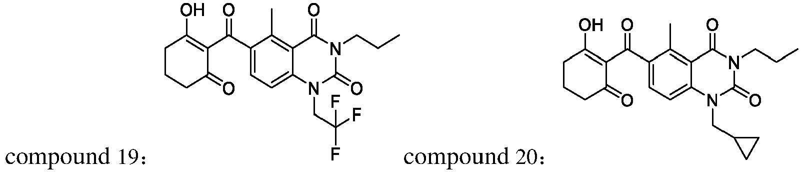

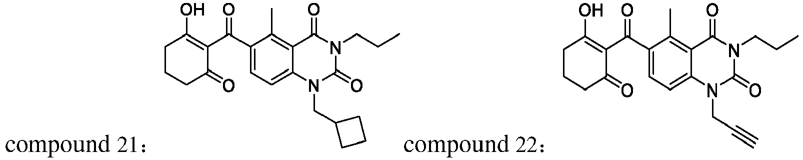

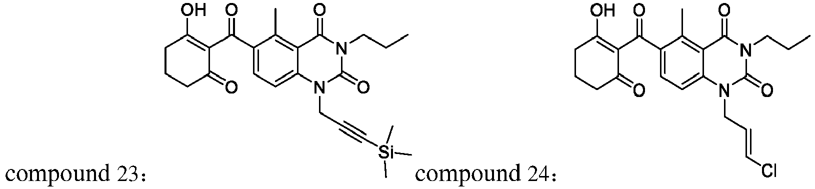

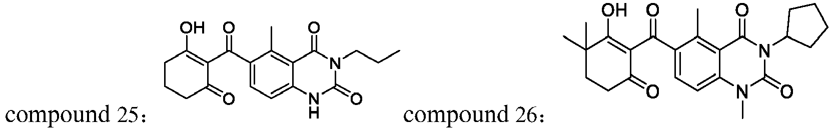

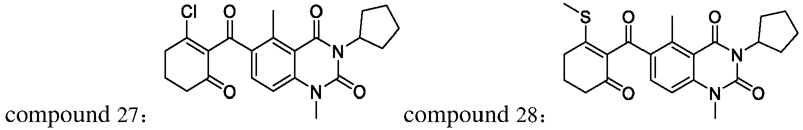

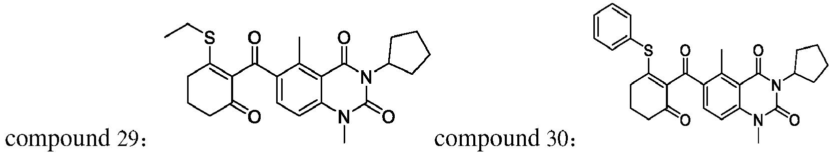

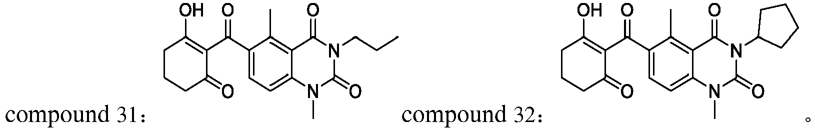

4. A method for preparing the triketone compounds of any one of claims 1 to 3, which comprises:
(1) Reacting the compounds with the structure shown in the formula (II-1) with sulfoxide chloride and the compound with the structure shown in the formula (II-2) in sequence to obtain the compound with the structure shown in the formula (II-3);
(2) Under the rearrangement reaction condition, the compound with the structure shown in the formula (II-3) are contacted with catalysts in the presence of alkalis and solvents to obtain the compound with the structure shown in the formula (II-4);
optionally, the method further comprises 1, 2, or 3 of the following steps in sequence:
(a) Carrying out halogenation reaction on the compound with the structure shown in the formula (II-4) to obtain the compound with the structure shown in the formula (II-5);
(b) Reacting the compound with the structure shown in the formula (II-5) with sodium mercaptide with the structure shown in the formula (II-6) to obtain the compound with the structure shown in the formula (II-7);
(c) Carrying out oxidation reaction on the compound with the structure shown in the formula (II-7) in the presence of peroxide with the structure shown in the formula (II-8) to obtain the compound with the structure shown in the formula (II-9); 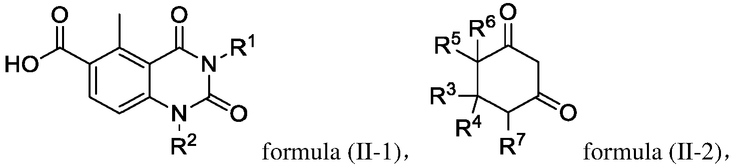 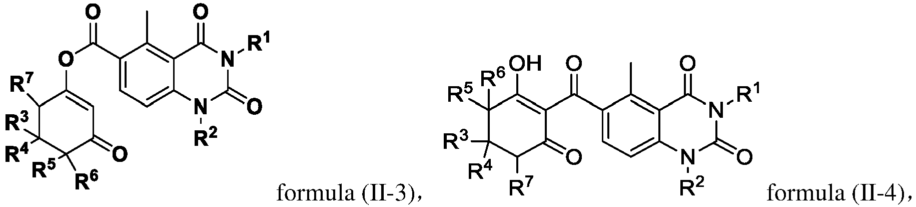 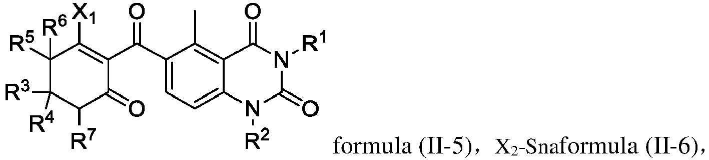 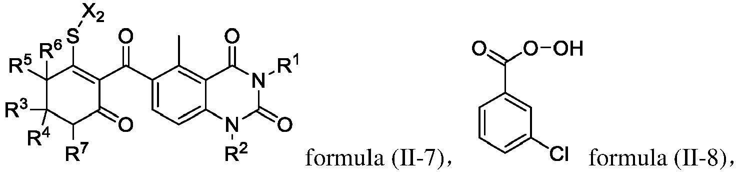 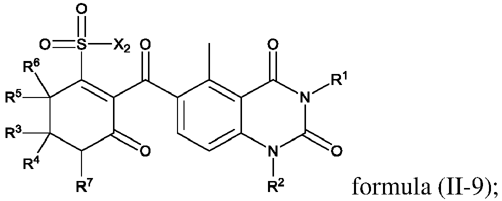
wherein,
X₁ in the compound with the structure shown in the formula (II-5) is halogen;
X2 in the compounds with the structure shown in the formula (II-6), the formula (II-7) and the formula (II-9) is selected from C₁₋₃alkyl and phenyl;
moreover, the remaining substituents involved in the formula (II-1), the formula (II-2), the formula (II-3), the formula (II-4), the formula (II-5), the formula (II-7), and the formula (II-9) are the same as those of the aforementioned compounds of any one of claims 1 to 3.

5. The methods according to claim 4, wherein, in step (2), the contacting conditions include: the reaction temperature is 0-100°C; the reaction time is 0.5-36 h.

6. The methods according to claim 4 or 5, wherein, in step (2), the catalysts are at least one selected from sodium cyanide, potassium cyanide, acetone cyanohydrin, trimethylsilyl cyanide, 1,2,4-triazole, and 1,2,4-benzotriazole;
preferably, the alkalis are at least one selected from potassium carbonate, sodium carbonate, cesium carbonate, triethylamine, and pyridine;
preferably, the solvents are selected at least one from dichloromethane, trichloromethane, dichloroethane, acetonitrile, toluene, tetrahydrofuran, and benzene.

7. The use of the triketone compounds according to any one of claims 1 to 3 for controlling weeds; preferably, the weeds are grow in wheat fields and/or peanut fields;
preferably, the weeds are at least one selected from *Echinochloa crusgalli, Setaria viridis, Digitaria sanguinalis, Amaranthus tricolor, Chenopodium album, Abutilon theophrasti, Aegilops squarrosa, Avena fatua, Bromus japonicus, Alopecurus aequalis, Alopecurus japonicus, Sclerochloa dura, Polypogon fugax, Beckmannia syzigachne, Galium aparine, Malachium aquaticum, Veronica didyma, Cardamine hirsuta, Polygonum lapathifolium, Stellaria alsine* and *Vicia gigantea.*

8. The use according to claim 7, wherein, the triketone compounds are used in an amount of 10 to 400 g/ha.

9. A herbicide, which comprises active ingredients and adjuvants, wherein, the active ingredients comprise at least one selected from the triketone compounds of any one of claims 1 to 3;
preferably, the active ingredients are present in an amount of 1 to 99.9 wt%.

10. The herbicide according to claim 9, wherein, the dosage form of the herbicide are at least one selected from emulsifiable concentrate, suspension, wettable powder, powder, granule, aqueous solution, mother liquor and mother powder.
